# EUROPEAN PATENT APPLICATION

(11) **EP 2 130 831 A1**
(43) Date of publication of application: **09.12.2009**
(21) Application number: 08010364.1
(22) Date of filing: 06.06.2008
(51) Int. Cl.: C07D 471/04, C12P 17/18, A61K 31/4738, A61P 35/00

(54) **CDC25 inhibitors**

(71) Applicant: InterMed Discovery GmbH, 44227 Dortmund (DE)
(72) Inventor: Nussbaum, Franz, 40219 Düsseldorf (DE); Ebbinghaus, Andrea, 42369 Wuppertal (DE); Mayer-Bartschmid, Anke, 42489 Wülfrath (DE); Zitzmann, Werner, 51381 Leverkusen (DE); Wiese, Welf-Burkhard, 40764 Langenfeld (DE); Stadler, Marc, 67150 Niederkirchen (DE); Anlauf, Sonja, 42115 Wuppertal (DE)
(74) Representative: Brosch, Oliver

(57) **Abstract**

The invention relates to pyridoquinolones, their synthesis and pharmaceutical use. The pyridoquinolones are useful as CDC25 inhibitors.

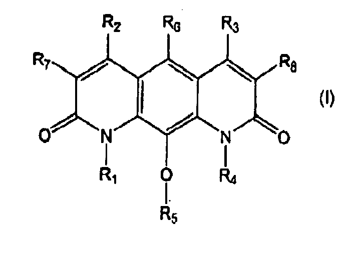

## Description

The invention relates to pyridoquinolones, to their use in medicaments, especially for the treatment of cancer. The pyridoquinolones are useful as CDC25 inhibitors.

The search for new compounds which are specifically active against enzymes controlling the cell division cycle has seen a lot of progress during the last years. Enzymes, which regulate the entry of the cell into mitotic M phase of the cell cycle are of particular interest as drug targets.

In eucaryotic cells, entry into M phase is initiated by activation of MPF (M Phase Promoting Factor) which consists of a catalytic sub-unit, CDC2 (Cell Division Cycle 2; synonym: CDK1) and a regulatory sub-unit, cyclin B (Morgan D. Annu. Rev. Cell Dev. Biol. 1997, 13, 261; Pavletich N. P. Mol. Biol. 1999, 287, 821).

In late prophase, the CDC2/cyclin B complex is inactive, due to the phosphorylation of two amino acid residues of CDC2, which are located just at the border of the kinase ATP-binding pocket. Activity of MPF results from the dephosphorylation of these inhibitory sites by a dual specificity phosphatase, the CDC25 phosphatase (Eckstein J. Gene Ther. Mol. Biol. 1998, 1, 707; Nilsson, I. Hoffmann in Progress in cell cycle research; L. Meijer, A. Jezequel, B. Ducommun, Eds.; Plenum: New York, 2000; Vol. 4, pp 107-114).

The CDC25 phosphatases (in the following also referred to as "CDC25") are key regulators of cell cycle progression and do play a central role in the checkpoint response to DNA damage- They dephosphorylate and activate cyclin-dependent kinases (CDKs) that, in association with their cyclin regulatory subunits, control progression at various stages of the cell-cycle. CDC25s belong to the dual-specificity phosphatase family: they dephosphorylate a threonine and a tyrosine residue of the same protein substrate (CDC2, also known as CDK1).

There are three CDC25 (A, B and C) genes in humans, encoding for three proteins and a large family of isoforms generated by alternative splicing and post-translational modifications. CDC25A is involved in the control of the G1/S transition by dephosphorylating and thus activating CDK2/cyclin E and cyclin A complexes. It has also been shown to be involved in the control of the progression into mitosis. CDC25B and C mainly regulate the progression at the G2/M transition and mitosis (Contour-Galcera MO. Pharmacol Ther. 2007, 115(1), 1-12). All CDC25 sub-types do control cell cycle propagation at different but always essential steps. Their respective overexpression has been reported from many different types of human tumors and it has been correlated with bad prognosis.

Specificity for one of the three human CDC25 phosphatases is still a matter of debate. Most of the CDC25 inhibitors identified yet do not differentiate inbetween the CDC25 sub-types. It is believed that a broad CDC25 inhibitory effect is a more efficient way to control cell proliferation through the targeting of multiple cell cycle essential steps (Brezak et al. 2005).

Their inhibition (CDC25 sub-types) therefore represents a promising therapeutic approach in oncology. Through their unique substrate selectivity and their essential functions in cell cycle control, CDC25 phosphatases are considered attractive screening targets to identify new anti-mitotic compounds.

CDC25 inhibitors are known from the prior art. In addition to synthetic compounds, several natural compounds which exhibit activity against CDC25 have been reported in the literature. Among these are coscinosulfate (Loukaci A., I. Le Saout, M. Samadi, S. Leclerc, E. Damkiens, L. Meijer, C. Debbitus, M. Guyot Bioorg. Med. Chem. 2001, 9, 3049-3054), nocardione (Otani T., Y. Sugimoto, Y. Aoyagi, Y. Igarashi, T. Furumai, N. Saito, Y. Yamada, T. Asao, Oki T. J. Antibiot. 2000, 53, 337-344) and dysidiolide (Shirai R., M. Takahashi, K. Dodo, K. Koga, Y. Hashimoto in Poster, PC-107, AFMC International Medicinal Chemistry Symposium/AIMECS 2001, Brisbane, Australia), which all inhibit CDC25 at micromolar levels.

However, the properties of the known CDC25 inhibitors are not satisfactory in every respect and thus, there is a demand for further CDC25 inbibitors.

It is an object of the invention to provide compounds that have advantages over the compounds of the prior art. The compounds should effectively inhibit CDC25 at comparatively low doses and should be useful as pharmaceutically active ingredients in medicaments, particularly in the treatment of cancer.

This object has been solved by the subject-matter of the patent claims.

It has been surprisingly found that certain pyridoquinolones exhibit CDC25 inhibitory activity.

A vast number of pyridinoquinones is known from the prior art. For example, pyridoquinones of the type of diazaquinomycin are known as antibacterial and anticancer agents (Kitasoto Institute, Omura S., H. Tanaka, K. Tsuzuki, Y. Murata, Kitasato Institute, Japan, 1988, JP 630 79 830) and several other derivatives of diazaquinomycin (dihydrodiazanathracenes, aza- and diaza-anthraquinonic derivatives) are said to exhibit antitumor activity (Avendano C., M. A. Alonso, M. Espada, D. Garcia-Gravalos, Universidad Complutense de Madrid, Spain, 1993, EP 0 574 195; Avendano Lopez C., D. Garcia Gravalos, Universidad Complutense de Madrid, Spain, 1995, WO 1995123145).

A large variety of pyridoquinolones are said to be useful in the treatment of hormone dependent diseases including various explicit hormone dependent cancers such us prostate and breast cancer (Ligand Pharmaceuficals: Jones T.K. et al., Ligand Pharmaceuticals, WO 1996/19458; Edwards J.P. et al., Ligand Pharmeceuticals, WO 1997/49709; Higuchi R. et al., Ligand Pharmaceuticals, WO 2001116139; Zhi, L. et al., Ligand Pharmaceuticals, WO 2002/066475).

Pyridoquinones as well as pyridoquinolones that exhibit CDC25 inhibitory activity, however, have not been reported in the literature.

A first aspect of the invention relates to compounds of general formula (1) 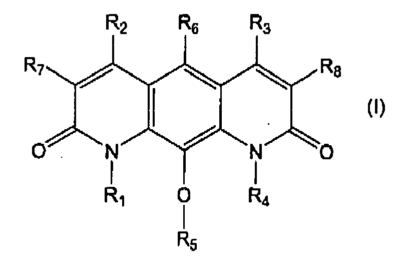 wherein
R₁ is -H, -C₁₋₆alkyl, -C(=O)-C₁₋₆-alkyk, -C(=O)-O-C₁₋₈-alkyl, -C(=O)-NH-C₁₋₈alkyl, -C(=O)-N(C₁₋₈-alkyln, -F, -Cl, -Br, -I, -NO₂, -CN, -CHO, -CO₂H, -OH, -O-G₁₋₈-alkyl, -O-C(=O)-C₁₋₈-alkyl. -SH, -S-C₁-₈-alkyf, -NH₂, -NH-C₁₋₈-alkyl, -NH-C(O)-C₁₋₈-alkyl, or -N(C₁₋₈-alkyl)₂; preferably -H, or -C₁₋₈-alkyl; more preferably -H, -CH₃ or -CF₃;
R₂ and R₃ are independently of each other -H, -C₁-₈-alkyl, -phenyl, -C₁₋₈-alkyl-phenyl, - C(=O)-C₁₋₈-alkyl, -C(=O)-O-C₁₋₈-alkyl, -C(=O)-NH-C₁₋₈-alkyl, -C(=O)-N(C₁₋₈-alkyl)₂, -F, - Cl, -Br, -I, -NO₂, -CN, -CHO, -CO₂H, -OH, -O-C₁₋₈-alkyl, -O-phenyl, -O-C₁₋₈-alkyl-phenyl, -O-C(=O)-C₁₋₈-alkyl, -SH, -S-C₁₋₈-alkyl, -NH₂, -NH-C₁-₈-alkyl, -NH-C(=O)-C₁₋₈-alkyl, or - N(C₁₋₈-alkyl)₂; preferably-C₁₋₈-alkyl; more preferably-CH₃, -CH₂CH₃, -CH(CH₃)₂, - CH₂CH₂CH₃, -CH₂OH, or -CF_{3;}
R₄ is -H, -C₁₋₈-alkyl, -phenyl, -C₁₋₈-alkyl-phenyl, -C(=O)-C₁₋₈-alkyl, -C(=O)-O-C₁₋₈-alkyl, - C(=O)-NH-C₁-₈-alkyl, -C(=O)-N(C₁₋₈-alkyl)₂, -F, -Cl, -Br, -I, -NO₂, -CN, -CHO, -CO₂H, - OH, -O-C₁₋₈-alkyl, -O-C(=O)-C₁₋₈-alkyl, -SH, -S-C₁₋₈-alkyl, -NH₂, -NH-C₁₋₈-alkyl, -NH-C(=O)-C₁₋₈-alkyl, or -N(C₁₋₈-alkyl)₂; preferably -H or -OH; more preferably -H;
R₅ is -H, -C₁₋₈-alkyl, -phenyl, -C₁₋₈-alkyl-phenyl, -C(=O)-C₁₋₈-alkyl, -C(=O)-O-C₁₋₈-alkyl, - C(=O)-NH-C₁-₈-alkyl, or -C(=O)-N(C₁₋₈-alkyl)₂; preferably -H or -C₁₋₈-alkyl; more preferably -H, -CH₃ or -CF₃:
R₆ is -H, -C₁₋₆-alkyl, -phenyl,-C₁₋₆-alkyl-phenyl, -C(=O)-C₁₋₈alkyl, -C(=O)-O-C₁₋₈-alkyl, - C(=O)-NH-C₁₋₈-alkyl, -C(=O)-N(C₁₋₈-alkyl)₂, -F, -Cl, -Br, -I, -NO₂, -CN, -CHO, -CO₂H, - OH, -O-C₁₋₈-alkyl, -O-phenyl, -O-C₁₋₈-alkyl-phenyl, -O-C(=O)-C₁₋₈-alkyl, -SH, -S-C₁₋₈-alkyl, -NH₂, -NH-C₁₋₈-alkyl, -NH-C(=O)-C₁₋₈-alkyl, or -N(C₁₋₈-alkyl)₂: preferably -H, -F or - C₁₋₈-alkyl; more preferably -H, -F or -CF₃;
R₇ and R₈ are idenpendently of each other -H, -C₁₋₈-alkyl, -C(=O)-C₁₋₈-alkyl, -C(=O)-O-C₁₋₈-alkyl, -C(=O)-NH-C₁₋₈-alkyl, -C(=O)-N(C₁₋₈-alkyl)₂, -F, -Cl, -Br, -I, -NO₂, -CN, -CHO, - CO₂H, -OH, -O-C₁₋₈-alkyl, -O-phenyl, -0-C₁₋₈-alkyl-phenyl, -O-C(=O)-C₁₋₈-alkyl, -SH, -S-C₁₋₈-alkyl, -NH₂, -NH-C₁₋₈-alkyl, -NH-C(=O)-C₁₋₈-alkyl, or -N(C₁₋₈-alkyl)₂; preferably -H, or -C₁₋₈-alkyl, more preferably -H or -CH₃;
or R₂ together with R₆ and/or R₂ together with R₇ and/or R₃ together with R₆ and/or R₃ together with R₈ independently of each other forms a five- to seven-membered saturated or unsaturated hydrocarbonaceous ring, where one or two C ring atoms are optionally replaced by heteroring atoms independently of each other selected from the group consisting of N, O and S, and where said hydrocarbonaceous ring is unsubstituted or contains one or two substituents independently of each other selected from the group consisting of -C₁₋₈-alkyl, -C(=O)-C₁₋₈-alkyl, -C(=O)-O-C₁₋₈-alkyl, -C(=O)-NH-C₁₋₈-alkyl, -C(=O)-N(C₁₋₈-alkyl)₂, -F, -Cl, -Br, -I, -NO₂, -CN, -CHO, -CO₂H, -OH, -O-C₁₋₈-alkyl, -O-C(=O)-C₁₋₈-alkyl, -SH, -S-C₁₋₈-a/kyl, -NH₂, -NH-C₁₋₈-alkyl, -NH-C(=O)-C₁₋₈-alkyl, or -N(C₁₋₈-alkyl)₂;
with the proviso
- that when R₅ is -H, R₆ is not -OH; and
- that R₁, R₂, R₃ and R₄ are not simultaneously -CH₃;
or the physiologically acceptable salts thereof,
for use as a medicament. It has been surprisingly found that the compounds of general formula (I) are suitable for pharmaceutical purposes, i.e., exhibit physiological and/or pharmacological effects.

According to general formula (I), R₆ is not -OH when R₅ is -H. Thus, the hydroquinone substructure is excluded. Diazaquinomycin B is a known antibiotic (cf. S. Omura et al., Journal of Antibiotics, 1985, 38(8), 1016). It seems that hydroquinones are oxidized *in vivo* to the corresponding quinones, which are not covered by general formula (I) either.

Further, according to general formula (I) R₁, R₂, R₃ and R₄ are not simultaneously -CH₃. 10-Hydroxy-1,4,6,9-tetramethylpyrido[3,2-g]quinotine-2,8(1H,9H)-dione (compound of example 6) has been reported to exhibit anti-neoplastic properties (Ojiri K., H. Suda, A. Okura, K. Kawamura, M. Okanishi, Banyu Pharmaceuticals, 1989, JP-B-28 03 182).

Preferably, R₁ is -H or -CH₃; R₄ is -H or -OH; and R₅ is -H.

Preferably, at least two residues selected from R₁, R₂, R₃ and R₄ are identical. In a preferred embodiment. R₂ and R₃ are identical and/or R₇ and R₈ are identical. In another preferred embodiment, R₁ and R₃ are identical. In still another preferred embodiment, R₁ and R₂ are identical. In yet another preferred embodiment, R₁, R₂ and R₃ are identical.

It has been surprisingly found that compounds of general formula (I), where R₅ is -H, i.e. anthroles, and where at least one of R₁ and R₄ is -H or -OH, exhibit a strong affinity against CDC25C.

The IC₅₀ value of these compounds with respect to CDC25C is significantly lower than the IC₅₀ value of known pyridoquinones, e.g., diazaquinomycin or compounds where R₁ and R₅ are together -CH₂- and form a five-membered saturated ring (i.e., nybomycin, deoxynybomycin, and the like).

Preferred compounds of general formula (I) are of general formula (II), (III), (IV) or (V):

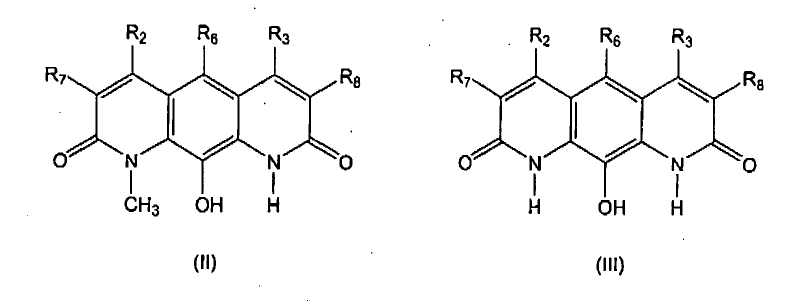

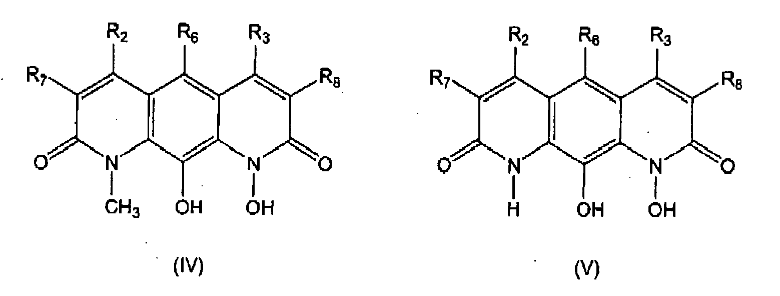

In preferred embodiments of the compounds according to general formulas (I) to (V), R₂ and R₉ are independently of each other -H, -OH, C₁₋₆-alkyl, or -C₁₋₈-alkyl-OH. Preferably, R₆ is -H, -F, -Cl, -Br, -C₁₋₈-alkyl, -CF₃. -O-C₁₋₈-alkyl, or -C₁₋₈-alkyl-OH. Preferably, R₇ and R₈ are idenpendently of each other -H, or -C₁₋₈-alkyl.

A further aspect of the invention relates to compounds of general formula (I) as defined above, with the proviso that
(i) when R₁, R₂ and R₃ are each -CH₃, and R₅, R₆, R₇ and R₈ are each -H; or
(ii) when R₁, R₂, R₃ and R₅ are each -CH₃, and R₆, R₇ and R₆ are each -H; or
(iii) when R₂, R₃ and R₅ are each -CH₃, and R₁, R₆, R₇ and R₈ are each -H;
   R₄ is not -H.

These three excluded compounds are known from the prior art. Demethylenedeoxynybomycin and 10-O-methyldemethylenedeoxynybomycin have been reported as synthetic intermediates in R.M. Forbis et al., JACS, 95:15,1973, 5003-13.

Particularly preferred compounds according to the invention are summarized in the table here below:

| no. | R₁ | R₂ | R₃ | R₄ | R₅ | R₆ | R₇ = R₈ |
|---|---|---|---|---|---|---|---|
| 1 | -H | -CH₃ | -CH₃ | -H | -H | -H | -H |
| 2 | -H | -CH₃ | -CH₂OH | -H | -H | -H | -H |
| 3 | -H | -CH₂OH | -CH₃ | -H | -H | -H | -H |
| 4 | -H | -CH₃ | -CH₂CH₂CH₃ | -H | H | -H | -H |
| 5 | -H | -CH₂CH₂CH₃ | -CH₃ | -H | -H | -H | -H |
| 6 | -H | -CH₂OH | -CH₂OH | -H | -H | -H | -H |
| 7 | -H | -CH₂OH | -CH₂CH₂CH₃ | -H | -H | -H | -H |
| 8 | -H | -CH₂CH₂CH₃ | -CH₂OH | -H | -H | -H | -H |
| 9 | -H | -CH₂CH₂CH₃ | -CH₂CH₂CH₃ | -H | -H | -H | -H |
| 10 | -H | -CH₃ | -CH₃ | -H | -H | -F | -H |
| 11 | -H | -CH₃ | -CH₂OH | -H | -H | -F | -H |
| 12 | -H | -CH₂OH | -CH₃ | -H | -H | -F | -H |
| 13 | -H | -CH₃ | -CH₂CH₂CH₃ | -H | -H | -F | -H |
| 14 | -H | -CH₂CH₂CH₃ | -CH₃ | -H | -H | -F | -H |
| 15 | -H | -CH₂OH | -CH₂OH | -H | -H | -F | -H |
| 16 | -H | -CH₂OH | -CH₂CH₂CH₃ | -H | -H | -F | -H |
| 17 | -H | -CH₂CH₂CH₃ | -CH₂OH | -H | -H | -F | -H |
| 18 | -H | -GH₂CH₂CH₃ | -CH₂CH₂CH₃ | -H | -H | -F | -H |
| 19 | -H | -CH₃ | -CH₃ | -H | -H | -CF₃ | -H |
| 20 | -H | -CH₃ | -CH₂OH | -H | -H | -CF₃ | -H |
| 21 | -H | -CH₂OH | -CH₃ | -H | -H | -CF₃ | -H |
| 22 | -H | -CH₃ | -CH₂CH₂CH₃ | -H | -H | -CF₃ | -H |
| 23 | -H | -CH₂CH₂CH₃ | -CH₃ | -H | -H | -CF₃ | -H |
| 24 | -H | -CH₂OH | -CH₂OH | -H | -he | -CF₃ | -H |
| 25 | -H | -CH₂OH | -CH₂CH₂CH₃ | -H | -H | -CF₃ | -H |
| 26 | -H | -CH₂CH₂CH₃ | -CH₂OH | -H | -H | -CF₃ | -H |
| 27 | -H | -CH₂CH₂CH₃ | -CH₂CH₂CH₃ | -H | -H | -CF₃ | -H |
| 28 | -H | -CH₃ | -CH₃ | -OH | -H | -H | -H |
| 29 | -H | -CH₃ | -CH₂OH | -OH | -H | -H | -H |
| 30 | -H | -CH₂OH | -CH₃ | -OH | -H | -H | -H |
| 31 | -H | -CH₃ | -CH₂CH₂CH₃ | -OH | -H | -H | -H |
| 32 | -H | -CH₂CH₂CH₃ | -CH₃ | -OH | -H | -H | -H |
| 33 | -H | -CH₂OH | -CH₂OH | -OH | -H | -H | -H |
| 34 | -H | -CH₂OH | -CH₂CH₂CH₃ | -OH | -H | -H | -H |
| 35 | -H | -CH₂CH₂CH₃ | -CH₂OH | -OH | -H | -H | -H |
| 36 | -H | -CH₂CH₂CH₃ | -CH₂CH₂CH₃ | -OH | -H | -H | -H |
| 37 | -H | -CH₃ | -CH₃ | -OH | -H | -F | -H |
| 38 | -H | -CH₃ | -CH₂OH | -OH | -H | -F | -H |
| 39 | -H | -CH₂OH | -CH₃ | -OH | -H | -F | -H |
| 40 | -H | -CH₃ | -CH₂CH₂CH₃ | -OH | -H | -F | -H |
| 41 | -H | -CH₂CH₂CH₃ | -CH₃ | -OH | -H | -F | -H |
| 42 | -H | -CH₂OH | -CH₂OH | -OH | -H | -F | -H |
| 43 | -H | -CH₂OH | -CH₂GH₂GH₃ | -OH | -H | -F | -H |
| 44 | -H | -CH₂CH₂CH₃ | -CH₂OH | -OH | -H | -F | -H |
| 45 | -H | -CH₂CH₂CH₃ | -CH₂CH₂CH₃ | -OH | -H | -F | -H |
| 46 | -H | -CH₃ | -CH₃ | -OH | -H | -CF₃ | -H |
| 47 | -H | -CH₃ | -CH₂OH | -OH | -H | -CF₃ | -H |
| 48 | -H | -CH₂OH | -CH₃ | -OH | -H | -CF₃ | -H |
| 49 | -H | -CH₃ | -CH₂CH₂CH₃ | -OH | -H | -CF₃ | -H |
| 50 | -H | -CH₂CH₂CH₃ | -CH₃ | -OH | -H | -CF₃ | -H |
| 51 | -H | -CH₂OH | -CH₂OH | -OH | -H | -CF₃ | -H |
| 52 | -H | -CH₂OH | -CH₂CH₂CH₃ | -OH | -H | -CF₃ | -H |
| 53 | -H | -CH₂CH₂CH₃ | -CH₂OH | -OH | -H | -CF₃ | -H |
| 54 | -H | -CH₂CH₂CH₃ | -CH₂CH₂CH₃ | -OH | -H | -CF₃ | -H |
| 55 | -CH₃ | -CH₉ | -CH₃ | -H | -H | -H | -H |
| 56 | -CH₃ | -CH₃ | -CH₂OH | -H | -H | -H | -H |
| 57 | -CH₃ | -CH₂OH | -CH₃ | -H | -H | -H | -H |
| 58 | -CH₃ | -CH₃ | -CH₂CH₂CH₃ | -H | -H | -H | -H |
| 59 | -CH₃ | -CH₂CH₂CH₃ | -CH₃ | -H | -H | -H | -H |
| 60 | -CH₃ | -CH₂OH | -CH₂OH | -H | -H | -H | -H |
| 61 | -CH₃ | -CH₂OH | -CH₂CH₂CH₃ | -H | -H | -H | -H |
| 62 | -CH₃ | -CH₂CH₂CH₃ | -CH₂OH | -H | -H | -H | -H |
| 63 | -CH₃ | -CH₂CH₂CH₃ | -CH₂CH₂CH₃ | -H | -H | -H | -H |
| 64 | -CH₃ | -CH₃ | -CH₃ | -H | -H | -F | -H |
| 65 | -CH₃ | -CH₃ | -CH₂OH | -H | -H | -F | -H |
| 66 | -CH₃ | -CH₂OH | -CH₃ | -H | -H | -F | -H |
| 67 | -CH₃ | -CH₃ | -CH₂CH₂CH₃ | -H | -H | -F | -H |
| 68 | -CH₃ | -CH₂CH₂CH₃ | -CH₃ | -H | -H | -F | -H |
| 69 | -CH₃ | -CH₂OH | -CH₂OH | -H | -H | -F | -H |
| 70 | -CH₃ | -CH₂OH | -CH₂CH₂CH₃ | -H | -H | -H | -H |
| 71 | -CH₃ | -CH₂CH₂CH₃ | -CH₂OH | -H | -H | -H | -H |
| 72 | -CH₃ | -CH₂CH₂CH₃ | -CH₂CH₂CH₃ | -H | -H | -F | -H |
| 73 | -CH₃ | -CH₃ | -CH₃ | -H | -H | -CF₃ | -H |
| 74 | -CH₃ | -CH₃ | -CH₂OH | -H | -H | -CF₃ | -H |
| 75 | -CH₃ | -CH₂OH | -CH₃ | -H | -H | -CF₃ | -H |
| 76 | -CH₃ | -CH₃ | -CH₂CH₂CH₃ | -H | -H | -CF₃ | -H |
| 77 | -CH₃ | -CH₂CH₂CH₃ | -CH₃ | -H | -H | -CF₃ | -H |
| 78 | -CH₃ | -CH₂OH | -CH₂OH | -H | -H | -CF₃ | -H |
| 79 | -CH₃ | -CH₂OH | -CH₂CH₂CH₃ | -H | -H | -CF₃ | -H |
| 80 | -CH₃ | -CH₂CH₂CH₃ | -CH₂OH | -H | -H | -CF₃ | -H |
| 81 | -CH₃ | -CH₂CH₂CH₃ | -CH₂CH₂CH₃ | -H | -H | -CF₃ | -H |
| 82 | -CH₃ | -CH₃ | -CH₃ | -OH | -H | -H | -H |
| 83 | -CH₃ | -CH₃ | -CH₂OH | -OH | -H | -H | -H |
| 84 | -CH₃ | -CH₂OH | -CH₃ | -OH | -H | -H | -H |
| 85 | -CH₃ | -CH₃ | -CH₂CH₂CH₃ | -OH | -H | -H | -H |
| 86 | -CH₃ | -CH₂CH₂CH₃ | -CH₃ | -OH | -H | -H | -H |
| 87 | -CH₃ | -CH₂OH | -CH₂OH | -OH | -H | -H | -H |
| 88 | -CH₃ | -CH₂OH | -CH₂CH₂CH₃ | -OH | -H | -H | -H |
| 89 | -CH₃ | -CH₂CH₂CH₃ | -CH₂OH | -OH | -H | -H | -H |
| 90 | -CH₃ | -CH₂CH₂CH₃ | -CH₂CH₂CH₃ | -OH | -H | -H | -H |
| 91 | -CH₃ | - CH₃ | -CH₃ | -OH | -H | -F | -H |
| 92 | -CH₃ | -CH₃ | -CH₂OH | -OH | -H | -F | -H |
| 93 | -CH₃ | -CH₂OH | -CH₃ | -OH | -H | -F | -H |
| 94 | -CH₃ | -CH₃ | -CH₂CH₂CH₃ | -OH | -H | -F | -H |
| 95 | -CH₃ | -CH₂CH₂CH₃ | -CH₃ | -OH | -H | -F | -H |
| 96 | -CH₃ | -CH₂OH | -CH₂OH | -OH | -H | -F | -H |
| 97 | -CH₃ | -CH₂OH | -CH₂CH₂CH₃ | -OH | -H | -F | -H |
| 98 | -CH₃ | -CH₂CH₂ CH₃ | -CH₂OH | -OH | -H | -F | -H |
| 99 | -CH₃ | -CH₂CH₂CH₃ | -CH₂CH₂CH₃ | -OH | -H | -F | -H |
| 100 | -CH₃ | -CH₃ | -CH₃ | -OH | -H | -CF₃ | -H |
| 101 | -CH₃ | -CH₃ | -CH₂OH | -OH | -H | -CF₃ | -H |
| 102 | -CH₃ | -CH₂OH | -CH₃ | -OH | -H | -CF₃ | -H |
| 103 | -CH₃ | -CH₃ | -CH₂CH₂CH₃ | -OH | -H | -CF₃ | -H |
| 104 | -CH₃ | -CH₂CH₂CH₃ | -CH₃ | -OH | -H | -CF₃ | -H |
| 105 | -CH₃ | -CH₂OH | -CH₂OH | -OH | -H | -CF₃ | -H |
| 106 | -CH₃ | -CH₂OH | -CH₂CH₂CH₃ | -OH | -H | -CF₃ | -H |
| 107 | -CH₃ | -CH₂CH₂CH₃ | -CH₂OH | -OH | -H | -CF₃ | -H |
| 108 | -CH₃ | -CH₂CH₂CH₃ | -CH₂CH₂CH₃ | -OH | -H | -CF₃ | -H |
| 109 | -H | -CH₃ | -CH₃ | -H | -H | -H | -CH₃ |
| 110 | -H | -CH₃ | -CH₂OH | -H | -H | -H | -CH₃ |
| 111 | -H | -CH₂OH | -CH₃ | -H | -H | -H | -CH₃ |
| 112 | -H | -CH₃ | -CH₂CH₂CH₃ | -H | -H | -H | -CH₃ |
| 113 | -H | -CH₂CH₂CH₃ | -CH₃ | -H | -H | -H | -CH₃ |
| 114 | -H | -CH₂OH | -CH₂OH | -H | -H | -H | -CH₃ |
| 115 | -H | -CH₂OH | -CH₂CH₂CH₃ | -H | -H | -H | -CH₃ |
| 116 | -H | -CH₂CH₂CH₃ | -CH₂OH | -H | -H | -H | -CH₃ |
| 117 | -H | -CH₂CH₂CH₃ | -CH₂CH₂CH₃ | -H | -H | -H | -CH₃ |
| 118 | -H | -CH₃ | -CH₃ | -H | -H | -F | -CH₃ |
| 119 | -H | -CH₃ | -CH₂OH | -H | -H | -F | -CH₃ |
| 120 | -H | -CH₂OH | -CH₃ | -H | -H | -F | -CH₃ |
| 121 | -H | -CH₃ | -CH₂CH₂CH₃ | -H | -H | -F | -CH₃ |
| 122 | -H | -CH₂CH₂CH₃ | -CH₃ | -H | -H | -F | -CH₃ |
| 123 | -H | -CH₂OH | -CH₂OH | -H | -H | -F | -CH₃ |
| 124 | -H | -CH₂OH | -CH₂CH₂CH₃ | -H | -H | -F | -CH₃ |
| 125 | -H | -CH₂CH₂CH₃ | -CH₂OH | -H | -H | -F | -CH₃ |
| 126 | -H | -CH₂CH₂CH₃ | -CH₂CH₂CH₃ | -H | -H | -F | -CH₃ |
| 127 | -H | -CH₃ | -CH₃ | -H | -H | -CF₃ | -CH₃ |
| 128 | -H | -CH₃ | -CH₂OH | -H | -H | -CF₃ | -CH₃ |
| 129 | -H | -CH₂OH | -CH₃ | -H | -H | -CF₃ | -CH₃ |
| 130 | -H | -CH₃ | -CH₂CH₂CH₃ | -H | -H | -CF₃ | -CH₃ |
| 131 | -H | -CH₂CH₂CH₃ | -CH₃ | -H | -H | -CF₃ | -CH₃ |
| 132 | -H | -CH₂OH | -CH₂OH | -H | -H | -CF₃ | -CH₃ |
| 133 | -H | -CH₂OH | -CH₂CH₂CH₃ | -H | -H | -CF₃ | -CH₃ |
| 134 | -H | -CH₂CH₂CH₃ | -CH₂OH | -H | -H | -CF₃ | -CH₃ |
| 135 | -H | -CH₂CH₂CH₃ | -CH₂CH₂CH₃ | -H | -H | -CF₃ | -CH₃ |
| 136 | -H | -CH₃ | -CH₃ | -OH | -H | -H | -CH₃ |
| 137 | -H | -CH₃ | -CH₂OH | -OH | -H | -H | -CH₃ |
| 138 | -H | -CH₂OH | -CH₃ | -OH | -H | -H | -CH₃ |
| 139 | -H | -CH₃ | -CH₂CH₂CH₃ | -OH | -H | -H | -CH₃ |
| 140 | -H | -CH₂CH₂CH₃ | -CH₃ | -OH | -H | -H | -CH₃ |
| 141 | -H | -CH₂OH | -CH₂OH | -OH | -H | -H | -H₃ |
| 142 | -H | -CH₂OH | -CH₂CH₂CH₃ | -OH | -H | -H | -CH₃ |
| 143 | -H | -CH₂CH₂CH₃ | -CH₂OH | -OH | -H | -H | -CH₃ |
| 144 | -H | -CH₂CH₂CH₃ | -CH₂CH₂CH₃ | -OH | -H | -H | -CH₃ |
| 145 | -H | -CH₃ | -CH₃ | -OH | -H | -F | -CH₃ |
| 146 | -H | -CH₃ | -CH₂OH | -OH | -H | -F | -CH₃ |
| 147 | -H | -CH₂OH | -CH₃ | -OH | -H | -F | -CH₃ |
| 148 | -H | -CH₃ | -CH₂CH₂CH₃ | -OH | -H | -F | -CH₃ |
| 149 | -H | -CH₂CH₂CH₃ | -CH₃ | -OH | -H | -F | -CH₃ |
| 150 | -H | -CH₂OH | -CH₂OH | -OH | -H | -F | -CH₃ |
| 151 | -H | -CH₂OH | -CH₂CH₂CH₃ | -OH | -H | -F | -CH₃ |
| 152 | -H | -CH₂CH₂CH₃ | -CH₂OH | -OH | -H | -F | -CH₃ |
| 153 | -H | -CH₂CH₂CH₃ | -CH₂CH₂CH₃ | -OH | -H | -F | -CH₃ |
| 154 | -H | -CH₃ | -CH₃ | -OH | -H | -CF₃ | -CH₃ |
| 155 | -H | -CH₃ | -CH₂OH | -OH | -H | -CF₃ | -CH₃ |
| 156 | -H | -CH₂OH | -CH₃ | -OH | -H | -CF₃ | -CH₃ |
| 157 | -H | -CH₃ | -CH₂CH₂CH₃ | -OH | -H | -CF₃ | -CH₃ |
| 158 | -H | -CH₂CH₂CH₃ | -CH₃ | -OH | -H | -CF₃ | -CH₃ |
| 159 | -H | -CH₂OH | -CH₂OH | -OH | -H | -CF₃ | -CH₃ |
| 160 | -H | -CH₂OH | -CH₂CH₂CH₃ | -OH | -H | -CF₃ | -CH₃ |
| 161 | -H | -CH₂CH₂CH₃ | -CH₂OH | -OH | -H | -CF₃ | -CH₃ |
| 162 | -H | -CH₂CH₂CH₃ | -CH₂CH₂CH₃ | -OH | -H | -CF₃ | -CH₃ |
| 163 | -CH₃ | -CH₃ | -CH₃ | -H | -H | -H | -CH₃ |
| 164 | -CH₃ | -CH₃ | -CH₂OH | -H | -H | -H | -CH₃ |
| 165 | -CH₃ | -CH₂OH | -CH₃ | -H | -H | -H | -CH₃ |
| 166 | -CH₃ | -CH₃ | -CH₂CH₂CH₃ | -H | -H | -H | -CH₃ |
| 167 | -CH₃ | -CH₂CH₂CH₃ | -CH₃ | -H | -H | -H | -CH₃ |
| 168 | -CH₃ | -CH₂OH | -CH₂OH | -H | -H | -H | -CH₃ |
| 169 | -CH₃ | -CH₂OH | -CH₂CH₂CH₃ | -H | -H | -H | -CH₃ |
| 170 | -CH₃ | -CH₂CH₂CH₃ | -CH₂OH | -H | -H | -H | -CH₃ |
| 171 | -CH₃ | -CH₂CH₂CH₃ | -CH₂CH₂CH₃ | -H | -H | -H | -CH₃ |
| 172 | -CH₃ | -CH₃ | -CH₃ | -H | -H | -F | -CH₃ |
| 173 | -CH₃ | -CH₃ | -CH₂OH | -H | -H | -F | -CH₃ |
| 174 | -CH₃ | -CH₂OH | -CH₃ | -H | -H | -F | -CH₃ |
| 175 | -CH₃ | -CH₃ | -CH₂CH₂CH₃ | -H | -H | -F | -CH₃ |
| 176 | -CH₃ | -CH₂CH₂GH₃ | -CH₃ | -H | -H | -F | -CH₃ |
| 177 | -CH₃ | -CH₂OH | -CH₂OH | -H | -H | -F | -CH₃ |
| 178 | -CH₃ | -CH₂OH | -CH₂CH₂CH₃ | -H | -H | -F | -CH₃ |
| 179 | -CH₃ | -CH₂CH₂CH₃ | -CH₂OH | -H | -H | -F | -CH₃ |
| 180 | -CH₃ | -CH₂CH₂CH₃ | -CH₂CH₂CH₃ | -H | -H | -F | -CH₃ |
| 181 | -CH₃ | -CH₃ | -CH₃ | -H | -H | -CF₃ | -CH₃ |
| 182 | -CH₃ | -CH₃ | -CH₂OH | -H | -H | -CF₃ | -CH₃ |
| 183 | -CH₃ | -CH₂OH | -CH₃ | -H | -H | -CF₃ | -CH₃ |
| 184 | -CH₃ | -CH₃ | -CH₂CH₂CH₃ | -H | -H | -CF₃ | -CH₃ |
| 185 | -CH₃ | -CH₂CH₂CH₃ | -CH₃ | -H | -H | -CF₃ | -CH₃ |
| 186 | -CH₃ | -CH₂OH | -CH₂OH | -H | -H | -CF₃ | -CH₃ |
| 187 | -CH₃ | -CH₂OH | -CH₂CH₂CH₃ | -H | -H | -CF₃ | -CH₃ |
| 188 | -CH₃ | -CH₂CH₂CH₃ | -CH₂OH | -H | -H | -CF₃ | -CH₃ |
| 189 | -CH₃ | -CH₂CH₂CH₃ | -CH₂CH₂CH₃ | -H | -H | -CF₃ | -CH₃ |
| 190 | -CH₃ | -CH₃ | -CH₃ | -OH | -H | -H | -CH₃ |
| 191 | -CH₃ | -CH₃ | -CH₂OH | -OH | -H | -H | -CH₃ |
| 192 | -CH₃ | -CH₂OH | -CH₃ | -OH | -H | -H | -CH₃ |
| 193 | -CH₃ | -CH₃ | -CH₂CH₂CH₃ | -OH | -H | -H | -CH₃ |
| 194 | -CH₃ | -CH₂CH₂CH₃ | -CH₃ | -OH | -H | -H | -CH₃ |
| 195 | -CH₃ | -CH₂OH | -CH₂OH | -OH | -H | -H | -CH₃ |
| 196 | -CH₃ | -CH₂OH | -CH₂CH₂CH₃ | -OH | -H | -H | -CH₃ |
| 197 | -CH₃ | -CH₂CH₂CH₃ | -CH₃OH | -OH | -H | -H | -CH₃ |
| 198 | -CH₃ | -CH₂CH₂CH₃ | -CH₂CH₂CH₃ | -OH | -H | -H | -CH₃ |
| 199 | -CH₃ | -CH₃ | -CH₃ | -OH | -H | -F | -CH₃ |
| 200 | -CH₃ | -CH₃ | -CH₂OH | -OH | -H | -F | -CH₃ |
| 201 | -CH₃ | -CH₂OH | -CH₃ | -OH | -H | -F | -CH₃ |
| 202 | -CH₃ | -CH₃ | -CH₂CH₂CH₃ | -OH | -H | -F | -CH₃ |
| 203 | -CH₃ | -CH₂CH₂CH₃ | -CH₃ | -OH | -H | -F | -CH₃ |
| 204 | -CH₃ | -CH₂OH | -CH₂OH | -OH | -H | -F | -CH₃ |
| 205 | -CH₃ | -CH₂OH | -CH₂CH₂CH₃ | -OH | -H | -F | -CH₃ |
| 206 | -CH₃ | -CH₂CH₂CH₃ | -CH₂OH | -OH | -H | -F | -CH₃ |
| 207 | -CH₃ | -CHzCH₂CH₃ | -CH₂CH₂CH₃ | -OH | -H | -F | -CH₃ |
| 208 | -CH₃ | -CH₃ | -CH₃ | -OH | -H | -CF₃ | -CH₃ |
| 209 | -CH₃ | -CH₃ | -CH₂OH | -OH | -H | -CF₃ | -CH₃ |
| 210 | -CH₃ | -CH₂OH | -CH₃ | -OH | -H | -CF₃ | -CH₃ |
| 211 | -CH₃ | -CH₃ | -CH₂CH₂CH₃ | -OH | -H | -CF₃ | -CH₃ |
| 212 | -CH₃ | -CH₂CH₂CH₃ | -CH₃ | -OH | -H | -CF₃ | -CH₃ |
| 213 | -CH₃ | -CH₂OH | -CH₂OH | -OH | -H | -CF₃ | -CH₃ |
| 214 | -CH₃ | -CH₂OH | -CH₂CH₂CH₃ | -OH | -H | -CF₃ | -CH₃ |
| 215 | -CH₃ | -CH₂CH₂CH₃ | -CH₂OH | -OH | -H | -CF₃ | -CH₃ |
| 216 | -CH₃ | -CH₂CH₂CH₃ | -CH₂CH₂CH₃ | -OH | -H | -CF₃ | -CH₃ |

The numbers of the individual compounds in the above table are not to be confused with the numbers of the individual compounds exemplified in the experimental section.

Particularly preferred compounds of formula (I) are selected from the group consisting of
10-hydroxy-4-(hydroxymethyl)-1,6-dimethylpyndo[3.2-g]quinoline-2.8(1H,9H)-dione;
10-hydroxy-3,7-dimethyl-4,6-dipropylpyrido[3,2-g]quinoline-2,8(1H,9H)-dione; and
1,10-dihydroxy-4,6,9-trimethylpyrido[3,2-g]quinoline-2,8(1H,9H)-dione
and the physiologically acceptable salts thereof.

The invention also relates to 10-hydroxy-1,4,6-trimethylpyrido[3,2-g]quinoline-2,8(1H,9H)-dione for use as medicament.

For the purpose of the specification, "alkyl" refers to a saturated or unsaturated, linear or branched and/or cyclic hydrocarbon which is unsubstituted or substituted with one, two or three substituents independently of each other selected from the group consisting of -F, -Cl, -Br, -1, -OH, -OCH₃, -OCH₂CH₃, -OCOCH₃, -CN, -NO₂, -NH₂, -CHO and -CO₂H. Thus, the term "alkyl" encompasses "alkyl", "alkenyl" and "alkynyl" as well as "cycloalkyl", "cycloalkenyl" and "cycloalkynyl". Examples of preferred alkyl residues are methyl, ethyl, n-propyl, i-propyl, n-butyl, sec.-butyl, iso.-butyl, tert.-butyl, n-pentyl, n-hexyl, n-heptyl and n-octyl. Examples of preferred substituted alkyl residues are hydroxymethyl, hydroxyethyl, hydroxypropyl, fluoromethyl, difluoromethyl, trifluoromethyl, acetoxy methyl and acetoxy ethyl. Examples of preferred alkenyl residues include vinyl, allyl and butadienyl. Examples of preferred alkynyl residues include etynyl and propargyl. A skilled person recognizes that a cyclic hydrocarbon requires the presence of at least 3 ring atoms. Examples of preferred cycloalkyl residues are cyclopropyl, cyclobutyl, cyclopentyl and cyclohexyl.

For the purpose of the specification, "phenyl" refers to a benzene moiety, unsubtituted or substituted with one, two or three substituents independently of each other selected from the group consisting of -F, -Cl, -Br, -I, -OH, -OCH₃, -OCH₂CH₃, -OCOCH₃, -CN, -NO₂, -NH₂, -CHO and -CO₂H; such as phenyl, o-fluorophenyl, m-fluorophenyl, p-fiuorophenyl, o-chlorophenyl, m-chlorophenyl, p-chlorophenyl, o-anisyl, m-anisyl, p-anisyl, and the like.

Physiologically acceptable salts of the compounds of general formula (I) include salts with physiologically acceptable acids as well as salts with physiologically acceptable bases. Physiologically acceptable acids include inorganic acids such as HCl, HBr, H₂SO₄, H₃PO₄ and the like; and organic acids such as formic acid, acetic acid, propionic acid, citric acid, maleic acid, malic acid, lactic acid, fumaric acid, and the like. Physiologically acceptable bases include ammonia, and organic amines.

The invention also relates to the stereoisomers of the compounds of general formula (I), such as enantiomers or diastereomers, tautomeric forms, salts, solvates such as hydrates, polymorphs, and the likes

A further aspect of the invention relates to pharmaceutical compositions comprising one or more compounds according to the invention as described above and a physiologically acceptable carrier. All preferred embodiments of the compound of general formula (I), e.g., the compounds of general formula (II), (III), (IV) or (V), also apply to the pharmaceutical composition according to the invention and thus, are not repeated here below.

The pharmaceutical composition according to the invention may be liquid, e.g. a solution, dispersion, suspension or emulsion; or solid, e.g. a powder, paste, gel, and the like.

Suitable physiologically acceptable carriers are known to the person skilled in the art. Suitable liquid carriers include water, ethanol and the like. Suitable solid carriers include typical pharmaceutical excipients, such as fillers, binders, glidants, disintegrants, and the like. In this regard it can be referred to, e.g., D.E. Bugay et al., Pharmaceutical Excipients, Informa Healthcare; 1 edition (December 1, 1998). In general, the pharmaceutical composition according to the invention may contain inert non-toxic pharmaceutically suitable auxiliaries, such as for example excipients, solvents, vehicles, emulsifiers and/or dispersants.

The following auxiliaries can be mentioned as examples: water, solid excipients such as ground natural or synthetic minerals (e.g. talcum or silicates), sugar (e.g. lactose), non-toxic organic solvents such as paraffins, vegetable oils (e.g. sesame oil), alcohols (e.g. ethanol, glycerol), glycols (e.g. polyethylene glycol), emulsifying agents, dispersants (e.g. polyvinylpyrrolidone) and lubricants (e.g. magnesium sulphate).

The relative weight ratio of the compound of general formula (I) and the physiologically acceptable carrier is preferably within the ratio of from 99.9 : 0.1 to 0.1 : 99.9.

A further aspect of the invention relates to pharmaceutical dosage forms containing the pharmaceutical composition according to the invention as described above. All preferred embodiments of the pharmaceutical composition according to the invention also apply to the pharmaceutical dosage form according to the invention and thus, are not repeated here below.

The pharmaceutical dosage forms according to the invention may be adapted, e.g., for systemic, local or topical administration. Systemic administration includes, e.g., intraveneous or oral administration.

The compounds according to the invention can exhibit non-systemic or systemic activity, wherein the latter is preferred. To obtain systemic activity the pharmaceutical dosage forms containing the active compounds can be administered, among other things, orally or parenterally, wherein oral administration is preferred. To obtain non-systemic activity the pharmaceutical dosage forms containing the active compounds can be administered, among other things, topically.

For parenteral administration, pharmaceutical dosage forms for administration to the mucous membranes (i.e. buccal, lingual, sublingual, rectal, nasal, pulmonary, conjunctival or intravaginal) or into the interior of the body are particularly suitable. Administration can be carried out by avoiding absorption (i.e. intracardiac, intra-arterial, intravenous, intraspinal or intralumbar administration) or by including absorption (i.e. intracutaneous, subcutaneous, percutaneous, intramuscular or intraperitoneal administration).

For the above purpose the pharmaceutical dosage forms containing the active compounds can be administered per se or in pharmaceutical dosage forms (administration forms).

Suitable pharmaceutical dosage forms for oral administration are, inter alia, normal and enteric-coated tablets, capsules, coated tablets, pills, granules, pellets, powders, solid and liquid aerosols, syrups, emulsions, suspensions and solutions. Suitable pharmaceutical dosage forms for parenteral administration are injection and infusion solutions.

The active compound can be present in the pharmaceutical dosage forms in concentrations of from 0.001-100% by weight; preferably the concentration of the active compound should be 0.5-90% by weight, i.e. quantities which are sufficient to allow the specified range of dosage.

In the case of oral administration, tablets can of course also contain additives such as sodium citrate as well as additives such as starch, gelatin and the like. Flavour enhancers or colorants can also be added to aqueous preparations for oral administration.

For the obtainment of effective results in the case of parenteral administration it has generally proven advantageous to administer quantities of about 0.001 to 100 mg/kg, preferably about 0.01 to 1 mg/kg of body weight. In the case of oral administration the quantity is about 0.01 to 100 mg/kg, preferably about 0.1 to 50 mg/kg of body weight.

In spite of this, it can be necessary in certain circumstances to depart from the amounts mentioned, namely as a function of body weight, application route, individual behaviour towards the active component: manner of preparation and time or interval at which application takes place. It can for instance be sufficient in some cases to use less than the aforementioned minimum amount, while in other cases the upper limit mentioned will have to be exceeded. In the case of the application of larger amounts, it can be advisable to divide them into a plurality of individual doses spread through the day.

The percentages in the tests and examples which follows are, unless otherwise stated, by weight; parts are by weight. Solvent ratios, dilution ratios and concentrations reported for liquid/liquid solutions are each based on the volume.

The pharmaceutical dosage forms may exhibit, e.g., an immediate or a sustained release profile of the active compound contained therein.

The compounds of the invention are inhibitors of CDC25 phosphatases, and in particular inhibitors of CDC25A and/or CDC25B and/or CDC25C phosphatase, and can therefore be used for preparing a medicament intended to inhibit CDC25 phosphatases.

The compounds according to the invention exhibit an unforeseeable, useful pharmacological and pharmacokinetic activity spectrum. They are therefore suitable for use as medicaments for the treatment and/or prophylaxis of disorders in humans and animals. The compounds of the formulae are CDC25-inhibitors suitable for use in therapy of proliferative disorders such as e.g. cancer in the pharma and animal health sector.

The compounds are particularly useful for the treatment of cancer such as ovarian cancer or colon cancer, tumor growth and metastasis, as well as proliferative, non-malignant diseases (hyperplasias).

In general, the compounds according to the invention are useful to prepare a medicament intended to treat a disease chosen from the following diseases: tumorous proliferative diseases, and in particular cancer, non-tumorous proliferative diseases, parasitic diseases, viral infections, spontaneous alopecia, alopecia induced by exogenous products and radiation-induced alopecia.

The compounds according to the invention are useful as cytostatics. For the purpose of the specification, cytostatic agents are defined as substances which do inhibit cell growth and cell division. In many cases these agents are even able to kill mammalian cells efficiently. Therefore, they are used for the treatment of neoplastic diseases such as cancer and can be derived as natural products or semi- and fully-synthetic compounds. In some cases cytostatics are also used for the treatment of other diseases than cancer such as e.g. autoimmune disorders (e.g. Multiple Sclerosis and Rheumatoide Arthritis). The group of cytostatics is composed of e.g alkylating agents (such as e.g. cyclophosphamide; ifosfamide and mitomycin); DNA crosslinkers (such as e.g. cisplatin; satraplatin); DNA intercalating compounds (such as e.g. doxorubicin; doxifluridin; and mitoxantron); antibiotics (such as e.g. bleomycin; actinomycin D; and mitomycin); inhibitors of mitosis /tubulin binders (such as e.g. paclitaxel and dotaxel; vincristin and vinblastin); topoisomerase inhibitors (such as e.g. camptothecin and etoposide); antimetabolites (such as e.g. methotrexate; 5-fluorouracil; and cladribin); and other cytostatics (such as e.g. hydroxycarbamide). In clinical cancer applications these compounds are often used in combination with each other therapeutics.

Another approach for therapy of neoplastic disorders does aim on cell cycle intervention. For this purpose kinase families such as e.g. cyclin-dependent kinases (CDKs), aurora and polo-like kinases (PLK) have been thoroughly studied. Several agents aiming on cell cycle inhibition such as e.g. flavopiridol and roscovitine. Some of them such as e.g. flavopiridol have already reached the clinical phases, even though compounds from these classes do still suffer from lack of specificity and efficacy.

Cytostatic agents such as listed above might therefore successfully be used in combination with cell cycle blocking agents such as CDC25 inhibitors.

A further aspect of the invention relates to the use of the compounds as described above for the manufacture of a medicament for treating any of the above indications, particularly for cancer, and to a method for treating cancer comprising the administration of a physiologically effective amount of a compound as described above, respectively.

Specific examples of cancer to which the compounds of the present invention are applied include, but are not limited to, malignant melanoma, malignant lymphoma, lung cancer, esophageal cancer, gastric cancer, large-bowel cancer, rectal cancer, colon cancer, urinary tract tumor, gallbladder cancer, bile duct cancer, biliary tract cancer, breast cancer, liver cancer, pancreatic cancer, testis tumor, maxillary cancer, tongue cancer, lip cancer, oral cavity cancer, pharynx cancer, larynx cancer, ovarian cancer, uterine cancer, prostate cancer, thyroid cancer, brain tumor, Kaposi's sarcoma, angioma, leukemia, polycythemia vera, neuroblastoma, retinoblastoma, myeloma, bladder tumor, sarcoma, osteosarcoma, myosarcoma, skin cancer, basal-cell carcinoma, cutaneous appendage tumor, skin metastatic cancer, and skin melanoma. In the present invention, a particularly preferable type of cancer to which the cancer suppressing agents are applied is brain tumor.

Preferably, the cancer is a carcinoma or malignant neoplasm selected from the group consisting of [C80]; [C73]; [C19]; [CO7]; [C12]; [C56]; [C01]; [C21.0]; [C16.0]; [C18.0]; [C21:2]; [C38.4]; [C44.9]; [C50.0]; [C76.0]: [C44.1]; [C11.1]; [C15.9]; [C16.9]; [C44.5]; [C51.2]; [C70.0]; [C70.9]; [C79.5]; [C06.9]; [C44.7]; [R52.1]; [C11.9]; [C21.1]; [C24.9]; [C30.0]; [C32.1]**;** [C34.9]; [C44.4]; [C60.1]; [C68.9]; [C75.1]; [C75.9]: [C10.4]; [C13.9]; [C16.1]; [C21.8]; [C24.0]; [C31.0]; [C41.1]; [C44.2]; [C44.3]; [C53.9]; [C54.2]; [C54.3]; [C54.9]; [C57.0], [C76.1]; [C00.5]; [C02.4]; [C16.3]; [C44-6]; [C50.9]; [C54.0]: [C57.3]; [C57.4]; [C67-5]; [CO5.0]; [C18.9]: [C22.9]; [C24.1]; [C34.0]; [C39.9]; [C69.4], [C72.9]; [C75.3]; [D04.9]; [DO5-1]; [Z51.1]; [C02.2]; [C05.1]; [C18.1]; [C18.2]; [C31.1]; [C31.2]; [C32.2]; [C39.0]; [C67.6]; [C71.1]; [C76.3]; [C14.8]; [C18.6]; [C18.7]; [C25.0]; [C25.2]; [C49.2]; [C51.0]; [C57.8]; [C67.0] [C67.1]; [C70.1]; [C71.2]; [C71.3]; [C75.4]; [C00.0]; [C00.3]; [C06.2]; [C09.0]; [C16.2]; [C18.4], [C22.0], [C25.1]; [C26.9]; [C31.9]; [C49.3]; [C71.4], [C74.0]; [C75.0]; [C76.2]; [C00.1]; [C00.4]**;** [C18.3]; [C18.5], [C25.4]; [C31.3]; [C34.1]; [C48.1]; [C49.5]; [C49.6]; [C63.7]; [C13.2]; [C17.3]; [C25.3]; [C51.1]; [C69.5], where the figure in brackets is the class according to ICD-10 of WHO, preferably in the version of 2008.

The compounds of general formula (I) can be synthesized by various routes. For example, the compounds can be prepared fully synthetically, starting from building blocks that are commercially available. Preferably, however, the compounds according to the invention are prepared by microorganisms or the products prepared by microorganisms are used as starting materials in the synthesis (semi-synthetic route). Thus, compounds of general formula (I) can be obtained from bacteria by fermentation, by semisynthetic derivatization of the compounds obtained by fermentation or by synthesis following previously published or new synthetic route- In other words, the compounds according to the invention can be, e.g., natural products, derivatives of these natural products or total synthetic analogs.

Some preferred methods for the preparation of compounds of general formula (I) are described here below:

### a) Fermentation / Isolation

The compounds according to the invention can be obtained from bacteria by fermentation of strains WS2260, G232, BA951, BS140 and BA859, belonging to the genus *Streptomyces,* extraction of resulting cultures with an organic solvent and subjecting the concentrated extract to a stepwise liquid-chromatographic purification.

The pyridoquinalones in question can be isolated from microorganisms according to this invention (strains WS2260, G232, BA951, BS140 and BA859) belonging to the genus *Streptomyces.* Other microbial strains of the genus *Streptomyces* (Strelitz, F. et al., Proc. Natl. Acad. Sci U.S.A., 1955, 41, 620-624; Rinehart K. L et al: J. Am. Chem. Soc. 1961, 83, 3729-3731; ibid. 1970, 92, 6994; Stadler M., T. Henkel, H. Müller, K. Weber, H. Schlecker; J. Chrom A 1998, 818, 187-195; Naganawa, H. et al., J. Antibiot., 1970, 23, 365-368; Forbis R. M., K. L. Rinehart, J. Am. Chem. Soc. 1973, 95, 5003-5013; Nadzan A. M., K. L. Rinehart, W. T. Sokolski J. Antibiot. 1977, 30, 523; Nadzan, A.M. et al., J. Am. Chem. Soc. 1977, 99, 4647-4654; Egawa, K. et al., Biol. Pharm. Bull., 2000, 23, 1036-1040) producing known pyridoquinolones have been decribed earlier.

A variety of procedures can be employed to ferment the obove mentioned streptomycetes, to isolate and purify the compounds from the fermentation broth, for example, by chromatographic adsorption procedures followed by elution with a suitable solvent, column chromatography, partition chromatography, and crystallisation from solvents and combinations thereof.

Suitable fermentation conditions, organic solvents for extraction and the chromatographic methods for isolation which can be used are known to the person skilled in the art or can be found in the literature (e.g. Stadler M, Bauch F, Henkel T, Mühlbauer A, Müller H, Spaltmann F, Weber K (2001). Arch. Pharm. Med. Chem. 334: 143-147).

### Fermentation

*Sfreptomyces* spp. WS2260, G232, BA951, BS140 and BA859 can be fermented in an aqueous nutrient medium under submerged aerobic conditions. Typically the microorganism is fermented in a nutrient medium containing a carbon source and a proteinaceous material. Preferred carbon sources include glucose, brown sugar, sucrose, glycerol, starch, com starch, lactose, dextrin, molasses, and the like. Preferred nitrogen sources include cottonseed flour, corn steep liquor, yeast, autolysed brewer's yeast with milk solids, soybean meal, cottonseed meal, com meal, milk solids, pancreatic digest of casein, distillers' solids, animal peptone liquors, meat and bone scraps, and the like. Combinations of these carbon and nitrogen sources can be used advantageously. Trace metals, for example. zinc, magnesium, manganese, cobalt, iron and the like need not be added to the fermentation medium since tap water and unpurified ingredients are used as medium components.

Production of compounds can be induced at any temperature conductive to satisfactory growth of the microorganisms between about 23° and 32° C and preferably at about 28 °C. Ordinarily, optimum production of compounds is obtained in about 2 to 6 days of fermentation, and preferably in about 4 to 5 days of fermentation. The fermentation broth normally remains weakly to moderately acidic during the fermentation, and advantageously the fermentation is terminated at pH of 4-4.5. The final pH is dependent, in part, on the buffers present, if any, and in part, on the initial pH of the culture medium. It is advantageously adjusted to about pH 6.5-7.5, and preferably 7.2, prior to sterilisation.

Production takes out in shake flask but also in solid media and stirred fermentors. When growth is carried out in shake flasks or large vessels and tanks, it is preferable to use the vegetative form, rather than the spore form, of the microorganism for inoculation to avoid a pronounced lag in the production of the compounds and the attendant inefficient utilisation of the equipment. Accordingly, it is desirable to produce a vegetative inoculum in an aqueous nutrient medium by inoculating this medium with an aliquot from a soil or a slant culture. When a young, active vegetative inoculum has thus been secured, it is transferred aseptically to other shake flasks or other suitable devices for fermentation of microorganisms. The medium in which the vegetative inoculum is produced can be the same as, or different from, that utilised for the production of compounds, as long as it is such that adequate growth of the microorganism is obtained.

In general, seeding of *Streptomyces* spp. strain(s) WS2260, G232, BA951, BS140 and BA859 and fermentation and the production of compounds in submerged aerobic fermentation in stirred vessels is utilised. The production is independent of used containers, fermentors and starter proceedings. The compounds can also be obtained by shake-flask culture. For large volume fermentations it is preferable to use a vegetative inoculum. The vegetative inoculum is prepared by inoculating small volume of culture medium with the spore form, mycelial fragments, or a lyophilised pellet of the organism. The vegetative inoculum is then transferred to a fermentation vessel where, after a suitable incubation time, compounds are produced in optimal yield.

As is customary in aerobic submerged culture process, sterile air is dispersed through the culture medium. For efficient growth of the organism, the volume of the air used is in the range of from about 0.25 to about 0.5 volume of air per volume of culture medium per minute (vvm). An optimum rate in a 10 L vessel is about 0.3 wm with agitation provided by conventional impellers rotating at about 240 rpm. Adding of small amount (i. e. 1 mL/i) of an antifoaming agent such as silicone to fermentations media is necessary if foaming becomes a problem. Preferred fermentation conditions and media are given in General Experimental Procedures

Compounds are present in the biomass of the fermentated *Streptomyces* spp. WS2260, G232, BA951, BS140 and BA859, as well as in the culture filtrate of the fermentation broth. The culture broth can be separated by filtering on a filter press, or by centrifugation and subsequent filtering.

### Extraction / Isolation

In the preferred recovery process, the compounds are extracted from the whole beer, from the mycelia or from extracts of the supernatant. The latter can be prepared by using adsorbant resins such as XAD, HP 20 or Bayer Lewapol. Column chromatography techniques, preferably over silica gels or modified silica gels, are used to perform the initial purification. Final purification of the compounds is preferably achieved by preparative medium-pressure liquid chromatography (MPLC) or High Performance Liquid Chromatography (HPLC).

Preferred solvents for the extraction are acetone, methanol or ethanol. Preferred solid carrier materials are finely divided silicas, aluminas or phenol-formaldehyde resins. Desorption is preferably carried out using 1. heptane or petroleum ether and 2. ether or t-butyl methyl ether. The preferred solvents in the subsequent liquid-liquid partition are successively 1. heptane or petroleum ether and 2. ether or t-butyl methylether.

In addition to methanol, it is also possible to choose ethanol, propanol, isopropanol, acetone, ethyl acetate, diethyl ether, t-butyl methyl ether, tetrahydrofuran, acetonitrile and other solvents known to the person skilled in the art for the extraction and the partial dissolution prior to application to solid carrier material. The pH value of the solvents can be modified by adding acids (trifluoroacetic acid, acetic and formic acid) or ammonium acetate, respectively.

Suitable solid carrier materials are, in addition to finely divided silicas and their modified variants such as, for example, modified silica gels (for example C2, C4, C8, C18, DIOL, phenyl and amino), also ground natural minerals, such as kaolins, clays, talc, chalk, quartz, montmorillonite and ground synthetic minerals, such as alumina, silicates or adsorber resins such as phenol-formaldehyde resins or polyamide resins.

For the successive desorption from the carrier material, it is also possible to choose successively 1. petroleum ether or heptane; 2. diethyl ether or ethyl acetate; 3. acetone or n-butanol or isobutanol, 4. methanol or ethanol or isopropanol. It is possible to use mixtures of the solvents with one another, in increasing solvent strength, too. The pH value of the solvents can be modified by adding acids (trifluoroacetic acid, acetic and formic acid) or ammonium acetate, respectively.

It is also possible to use other chromatographic methods such as gel permeation chromatography, countercurrent chromatography, or high speed counter current chromatography instead of the absorption chromatography described above.

Subsequent purification by preparative phase HPLC can also be carried out by the person skilled in the art using other stationary phases, such as RP8, phenyl, DIOL, C2, C4, C8 or amino. The mobile phase mixtures may also contain additional other acids (for example formic acid) or additional buffers (for example ammonium acetate). The organic component of the mobile phase can also be, for example, methanol in the case of separation on C18 and chloroform in the case of separation on silica gel.

To prepare the individual compounds according to the invention in pure form, a final purification via preparative HPLC separation can be carried out. Suitable separation systems are, alternatively, for example 1. solid phase = Nucleosil C18; mobile phase A = 0.1 % trifluoroacetic acid or formic acid or formate buffer or acetate buffer-containing water / B = 0.1% trifluoroacetic acid or formic acid or formate buffer or acetate buffer-containing acetonitrile or methanol; gradient of 0%-10% B initially increasing to 100% B over 45 - 90 minutes; 2. solid phase = silica gel; mobile phase: A = dichloromethane / B = methanol; gradient of 0% B initially increasing to 50% B over a period of 60 to 200 minutes.

The process is preferably carried out to obtain compounds of general formula (I) followed finally by liquid-chromatographic purification in a manner known to the person skilled in the art.

### b) Derivatisation

It will be clear to those having ordinary skill in this art that the compounds according to the invention can be obtained by semisynthetic approaches starting from compounds from bacterial fermentation. These standard methods for example can be deduced from the following publications: March's Advanced Organic Chemistry: Reaction, Mechanisms and Structure, 5th ed. by Michael B. Smith, Jerry March, Wiley-Interscience; 2001: Classics in Total Synthesis: Targets, Strategies, Methods by K. C. Nicolaou, E. J. Sorensen John Wiley & Son Ltd, 1996 and The Art and Science of Total Synthesis at the Dawn of the Twenty-First Century. Nicolaou KC, Vour-loumis D, Winssinger N, Baran PS, Angew Chem Int Ed Engl 2000, 39 (1): 44 -122. The following derivatisations are examples and the invention is not limited to them: N-protections, esterifications, acylations, alkylations etc..

### c) Total Synthesis

The compounds according to the invention are synthesised by methods which are known *per se* from the literature, as described in standard works on organic synthesis, for example Houben-Weyl, Methoden der organischen Chemie [Methods in Organic Chemistry], Georg Thieme-Venag. Stuttgart.

The preparation is carried out under reaction conditions which are known and suitable for the above-mentioned reactions. Other variants which are known *per se*, but not illustrated here in greater detail, may also be used.

If desired, the starting materials may be formed in situ, in such a way that they are not isolated from the reaction mixture but immediately reacted further to give the compounds of general formula (I).

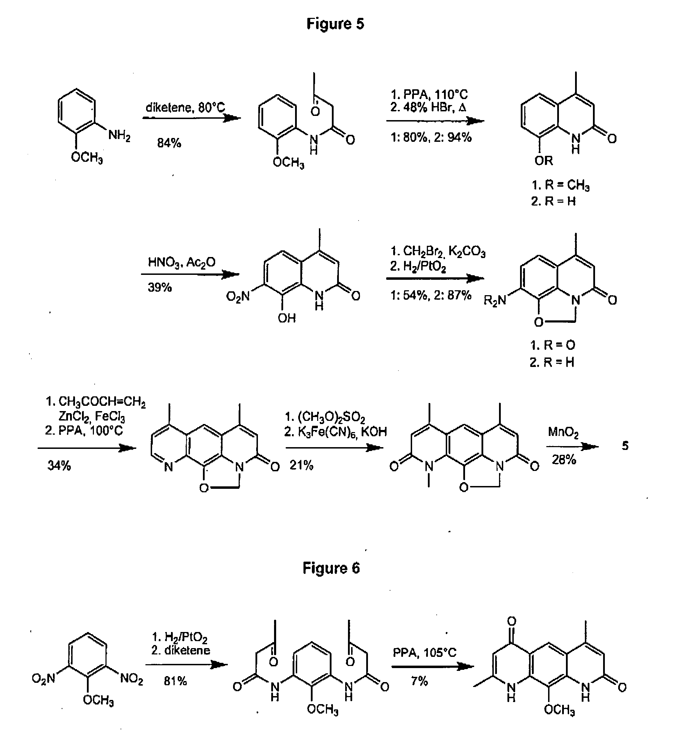

Synthetic routes towards this compound class have been elaborated and published previously for example by Rinehart (Forbis & Rinehart JACS 1973, 95:5003 - 5013). Compounds of the formula (I) can be synthesised following procedures starting from ortho-anisidin via a multistep synthesis as shown in the process in figure 5.
This synthesis yields the desired product in an overall yield of only 0.23%.

Attempts of the same authors of more straight foreward syntheses using a double Michael Friedel-Crafts reaction as outlined in figure 6 failed because the ring closing reaction yields only in the isomeric quinolonic compound which could not be rearranged to the desired substitution pattern.

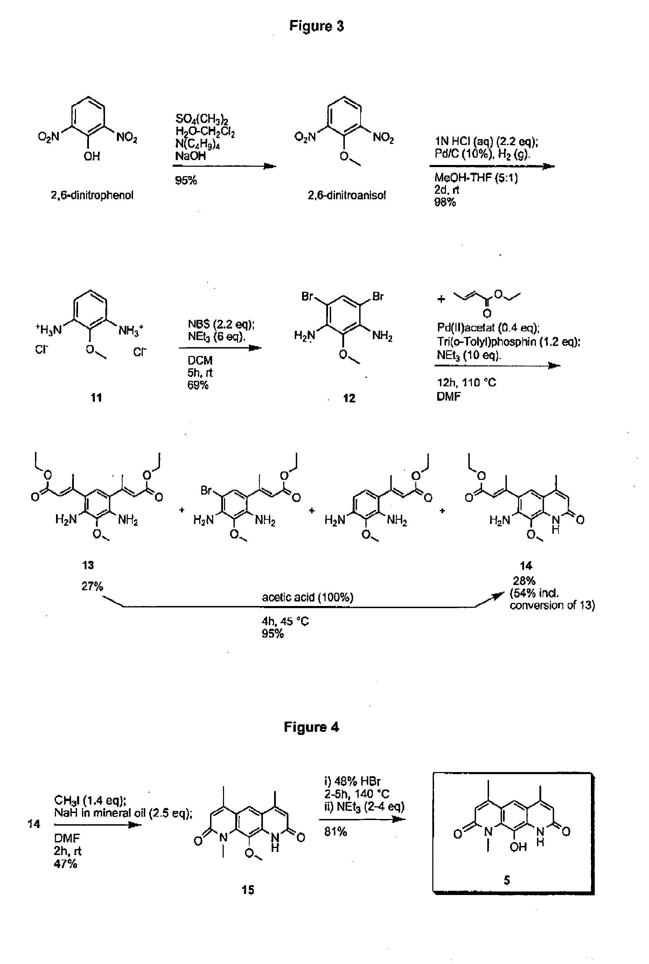

A new total synthetic route according to a procedure using a reaction under Heck conditions opens an efficient synthetic access towards the desired correct pseudo symmetric pyridoquinotones. This new synthesis, also following a double reaction approach, opens the way to compound 5 in a highly efficient way. The great advantage of this route is the possibility of large scale reactions in all synthetic steps and the high overall yield of about 8 %. In the key step an electron rich highly substituted aromatic system (12) is decorated with two new sustituents with a remarkable yield of 54 % as depicted in figure 3. Compound 14 is easily transformed into 5 within two steps shown in figure 4.

A further aspect of the invention relates to a microorganism deposited under the deposition number DSM 18699, DSM 18700, DSM 18701, DSM 18698 or DSM 18697 with DSMZ, Deutsche Sammlung von Mikroorganismen und Zellkulturen GmbH (German Collection of Microorganisms and Cell Cultures Ltd), Mascheroder Weg 1b, D-38124 Braunschweig, Germany.

### Molecular phylogeny

The five actinomycete strains producing compounds 1-10 were studied for their molecular phylogeny by comparison of their ribosomal DNA with those of the most similar strains from public databases, as descibed in detail by Stadler (J. Nat. Prod. 2007, 70: 246-252).

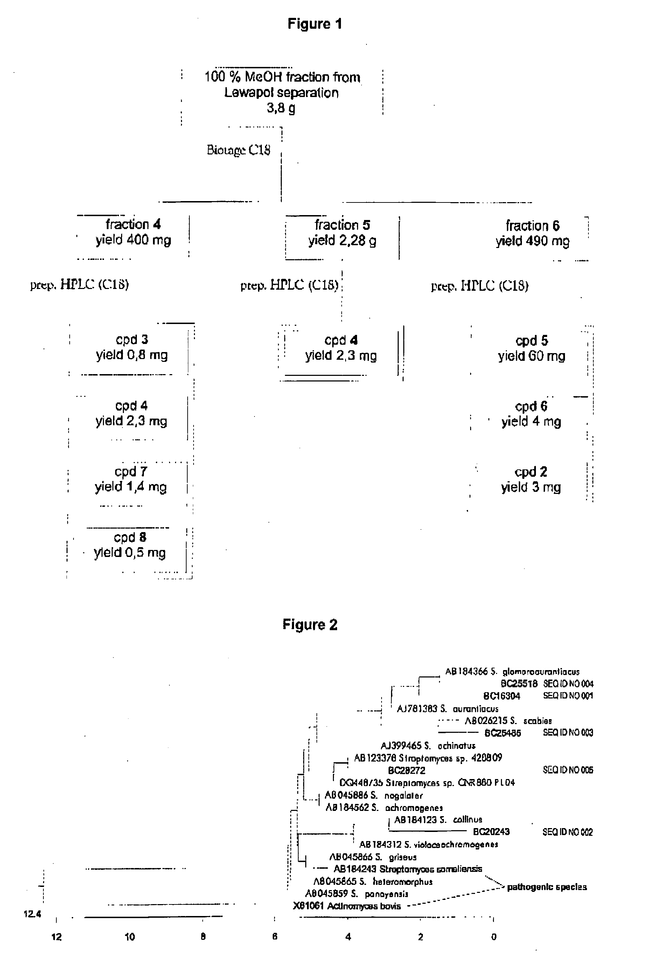

The main part of the 16S rRNA gene was amplified in a PCR using primers 41f and 1486r-P (unpublished) applying a standard thermal profile with an annealing temperature of 53°C. Amplification products were purified using Microspin S-200-HR Columns (Amersham Biosciences) using the protocol supplied by the manufacturer. Nucleotide sequences were obtained by cycle sequencing using the CycleReader Auto DNA Sequencing Kit (Fermentas), 5' IRD700 labeled primer 41f (custom synthesized by MWG Biotech), and the LI-COR 4200 Genetic Analyzer (LI-COR Inc. Lincoln, Nebraska, U.S.A.). A search for sequences similar to the ones determined was performed using FASTA as provided as on-line service by the European Bioinformatics Institute (http://www.ebi.ac.uk/fasta33/index.htmL). The sequences showing the best matches were obtained from the database to be used as input for the MegAlign module of the LASERGENE software (DNASTAR Inc, Madison, Wisconsin, USA).
About 400 bases of sequence were obtained from each of the five actinomycetes (sequence listing shown of the 16S rDNA). The sequences were used as FASTA input to search for similar sequences in the EMBL databank. All sequences were found to be closely (>97% similarity) related to 16S rRNA sequences of members of streptomycetes. A selection of these (table #1) as well as the 16S rDNA sequences of the risk group 2 organisms *Actinomyces bovis* (Acc. No. X81061) and *Streptomyces somaliensis* (Ace. No. AB184243) were used for making an alignment (Clustal method) and setting up a dendrogram (Fig. 2).

**Table #1**

| Accession no. | Genus | Species | Strain |
|---|---|---|---|
| AJ781383 | *Streptomyces* | *aurantiacus* | |
| AB 184366 | *Streptomyces* | *glomeroaurantiacus.* | |
| AB026215 | *Streptomyces* | *scabies* | |
| AB045859 | *Streptomyces* | *panayensis* | |
| AB123378 | *Streptomyces* | *sp*. | 428B09 |
| DQ448735 | *Streptomyces* | *sp*. | CNR880 PL04 |
| AB045886 | *Streptomyces* | *nogalater* | |
| AB045865 | *Streptomyces* | *heteromorphus* | |
| AB184562 | *Streptomyces* | *achromogenes* | |
| AJ399465 | *Streptomyces* | *echinatus* | |
| AB184923 | *Streptomyces* | *collinus* | |
| AB045866 | *Streptomyces* | *griseus* | |
| AB184312 | *Streptomyces* | *violaceochromogenes* | |

16S rDNA Sequences of *Streptomyces* spp. identified by FASTA searches performed in December of 2006, to be closely related to the 16S rDNA sequences of *Streptomyces* spp. strains BC16304, BC20243, BC25518, BC25486 and 8C28272.

Fig 2: Dendrogram of the 16S rDNA Sequences of the five samples, the closest relatives as found by FASTA searches and the pathogenic species *S. somalionsis. Actinomyces bovis* is included as an outgroup.

As inferred from molecular phylogeny, it was found that all five strains investigated belong to the genus *Streptomyces.* They were well separated from the only risk group 2 species (*S. somaliensis*). Thus, it can be concluded with certainty that the five isolates investigated are non-pathogenic *Streptomyces* species.

The following examples further illustrate the invention but are not to be construed as limiting its scope.

Characterisation and maintenance of Streptomyces spp.

**Culture media table**

| medium | ingredients |
|---|---|
| Yeast-Malt-Glucose (YMG) medium: | D-glucose 0.4%, malt extract 1%, yeast extract 0.4%, pH 7.2. |
| Q6 medium: | D-glucose 0.4%, glycerol 2%, cotton seed meal 1%, tap water, pH 7.2. |
| C medium: | D-glucose 1 %, yeast extract 1%, NZ amine (Sheffield Chemicals, Sheffield, U,.K., Lot ONA 20 2) 0.5%, soluble starch 2%, no pH adjustment |
| GS medium: | D-glucose 2%, deoiled soymeal (Soyamin 50 T, Degussa, Dilsseldorf, Germany) 2%, soluble starch 2%, calcium carbonate 0.5%, sodium chloride 0.25%, magnesium sulfate 0.05%, potassium dihydrogen phosphate 0.025%, pH adjustment to 6.5-6.8 |
| MC medium: | D-glucose 1%, yeast extract 0.5%, deoited soymeal (Soyamin 50 T) 1%, soluble starch 1%, sodium chloride 0.5%, calcium carbonate 0.3%, pH adjustment to 7.2 (0.1N sodium hydroxide solution). |
| MS medium: | Mannitol 2%, Soymeal defatted (Soyamin 50 T) 2%, calcium carbonate 0.3%, pH adjusted to 7.5. |

All strains are maintained at the Deutsche Stammsammlung fur Mikroorganismen und Zellkulturen, Mascheroder Weg 1b, D-38124 Braunschweig, Germany (DSMZ) under the given identifies

| strain | | origin | DSMZ registry | SEQ ID NO |
|---|---|---|---|---|
| BA 985 | Dr. O. Salcher (1985) at Bayer AG. Wuppertal | soil sample of unknown origin | BC25518 DSM 18699 | 004 |
| BA 951 | Dr. O. Salcher (1985) | soil sample of unknown origin | BC25486 DSM 18700 | 003 |
| BS140 | Dr. O. Salcher (1985) | soil sample collected in Germany | BC28272 . DSM 18701 | 005 |
| G232 | Dr. G. Vobis (1983) at the university of Marburg, Germany | soil sample collected in Australia and New Zealand | BC20243 DSM 18698 | 002 |
| WS2260 | Dr. O. Salcher (1985) | soil sample collected in Chile | BC16304 DSM 18697 | 001 |

By means of a bioassay-controlled fractionation, nine representatives of the structural class of pyridoquinolones and derivatives have been isolated.

### abbreviations

| | |
|---|---|
| ACN | acetonitrile |
| aq. | aqueous |
| DCM | dichloromethan |
| DMF | dimethylformamide |
| DMSO | dimethylsulfoxide |
| ELSD | electron light scattering detector |
| ESI | electro-spray ionisation |
| h | hour / hours |
| HPLC | high pressure liquid chromatography |
| LC/MS | liquid chromatography-coupled mass spectrometry |
| min. | minute(s) |
| MS | mass spectrometry |
| NMR | nuclear magnetic resonance spectrometry |
| RP | reverse phase (HPLC) |
| Rₜ | retention time (HPLC) |
| rt | room temperature |
| TFA | trifluoroacetic acid |
| UV | ultraviolet |
| UVNis | ultraviolet-visual |
| % of th. | % of theoretical yield |

### General Experimental Procedures

Chemicals were obtained in analytical grade from Merck (Darmstadt, Germany) or Sigma Aldrich (Deisenhofen, Germany). Analytical HPLC (Sys 1) was carried out on a HP 1100 (Hewlett Packard, Waldbronn, Germany) unit comprising a G 1312A binary pump system, a G 1315A diode array detector, a G 1316A column compartment, a G 1322A degaser and a G 1313A autoinjector, employing the following instrumental conditions: column Macherey & Nagel, Nucleodur 100-5 C18EC (125 X 4 mm); mobile phase A: 0.1 % TFA in water; mobile phase B: 0.1% TFA in methanol; flow: 1 mL/min; Gradient: start 100% A; 20 min: 100% B; 25 min: 100 % B; detection at 210 and 254 nm and with ELSD. ES1 HPLC-MS and ES1 high resolution mass spectrometry were recorded on HP 1100 coupled to a Micromass-LCT mass spectrometer (Micromass, Manchester, UK) using a waters symmetry column as stationary phase (50 X 2.1 mm; 3 µM); mobile phase A: 0.1% formic acid in water; mobile phase B: 0.1% formic acid in acetonitrile; gradient: start: 0 min 100% A; 1 min: 100% A; 5 main: 10% A; 6 min: 10% A; 7 min: 100% B; NMR spectra were recorded on a Bruker AVANCE600 Spectrometer, operating at 500.13 MHz proton frequency. All spectra were measured in DMSO-d_{β} solution at 302 K. The solvent peak was used as internal reference for both proton and carbon chemical shifts (δ_{H}: 2.50, δ_{C}: 39.5).

Preparative HPLC was performed at room temperature on a preparative HPLC system (Gilson Abimed, Ratingen, Germany), comprising Gilson Unipoint software, 306 binary pump system, 205 fraction collector, 119 UV-Vis detector, 806 manometric module, and 811C dynamic mixer, using different gradients and stationary phases as described below.

Fig. 1: Scheme for the isolation of examples 1 to 8 from the crude extracts of a fermentation of strain BS140 40 L scale

### Example 1: Fermentation of Streptomyces spp. strains

Seed culture: Two mL of a 10% glycerol culture of the strain WS2260 (and with the same procedure the strains G232, BA951, BS140 and BA985 were cultivated) are used to inoculate 1 L Erlenmeyer flasks containing 150 mL of sterile YMG medium and propagated on a rotary shaker at 28°C and 240 rpm for 72-96 h.

Fermentation of *Streptomyces* spp. in flask scale and HPLC profiling.- After inoculation from a well-grown YMG seed culture (2 mL inoculum per flask) were propagated in 1 L Erlenmeyer flasks containing 150 mL of MS medium or another production media as listed above and propagated on a rotary shaker at 28°C and 240 rpm for up to 142 h. During fermentation, daily samples were taken and analysed by HPLC. Albeit the metabolites were detected in all strains, different strain specific profiles and varying production rates of pyrridoquinolone patterns (compounds 1 - 9,) were observed. Out of the various culture media studied, MS medium was generally best suited for production, followed by GS and MC media. Production of compounds 1 - 9 was examplified below in order to obtain sufficient amounts for their biological and physico-chemical characterization. The process was therefore further optimized using MS medium and strain WS 2260 (large scale shake flask batch fermentation) (and BS140 (30 L scale stirring fermentor batch fermentation), respectively). Similar procedures as outlined below in examples 2-4, were also feasible for the other *Streptomyces* spp. to produce compounds 1 - 9 and/or similar compounds.

### Example 2: Isolation of compound 9 and 10 from shake cultures of Streptomyces WS2260

The pyridoquinolones of examples 9 and 10 were isolated from the combined mycelia prepared by fermenting 200 shake flasks (1 L) of WS2260 on a rotary shaker for 28°C at 240 RPM for 96 has follows: The culture broth was subjected to centrifugation (10 min at 4000 x g) and the fluid discarded for lack of the desired compounds. The dried mycelia (247 g) was extracted three times with 1 L methanol to afford 9 g of a crude extract. This crude extract was dissolved in 200 mL methanol and concentrated *in vacuo* together with 30 mL RP-18 silica gel (Macherey & Nagel; Düren, Germany, Polygoprep 1000-50-C18) . The dry powder was filled into a Biotage (Uppsala, Sweden) injection module and chromatographed on a Biotage KP-C18-WP column (15-30 µM, 300 A; 150x40 mm, mobile phase A: 0.1% TFA in water; mobile phase B: 0.1% TFA in acetonitrile; gradient: start: 100% A, 1 1 min: 100% A, 55 min: 100% B, 130 min: 100% B; flow 10 mL/min for the first 10 min and than 20 mL/min). The fraction eluting between 39 and 50 min (67 mg) was further separated on a Nucleosil 100-7 C18 preparative HPLC column (Machery & Nagel, 250x10 mm, 7 µm, mobile phase A: 0.1% TFA in water; mobile phase B: 0.1% TFA in acetonitrile; gradient: start: 5% B, 1 min: 5% B, 30 min: 80% B, 35 min: 80% B; flow: 10 mL/min). The fraction eluting between 24 and 25.5 min (7 mg) min was further separated on Sephadex LH 20 (500x15 mm, mobile phase: methanol, flow 2 mL/min) to afford each 1 mg of pure BAY 68-2820 (compound 9) and BAY 66-5836 (compound 10).

### Example 3: Isolation of compound 1 - compound 8 from the mycelia of a fermentation of Streptomyces sp. strain BS140

The compounds of examples 1-8 were isolated from the culture broth of strain BS140 fermented in a 40 L fermentor (constructed at Bayer Healthcare) containing 30 L of culture medium at 28°C and 240 RPM for 96 h in MS medium as follows: After centrifugation to separate the mycelium, the culture broth was adsorbed on Bayer (Leverkusen, Germany) Lewapol CA 9225 resin and the resin eluted stepwise with 4 L water, 4 L MeOH/water 1:1 and 4 L MeOH. The MeOH fraction was evaporated in vacuo to yield 3-8 g crude extract which was redissolved in MeOH and adsorbed on 30 mL RP18 silica gel (Macherey & Nagel Polygoprep 1000-50-C18) .The dry powder was filled into a Biotage injection module and chromatographed on Machery & Nagel Chromabond BT HPLC column (Nucleodur 100-20 C18ec, 130x40 mm, mobile phase A: 0.1% TFA in water; mobile phase B: 0.1% TFA in acetonitrile; gradient: start: 100% A, 11 min: 100% A, 55 min: 100% B, 130 min: 100% B; flow 10 mL/min for the first 10 min and thereafter 20 mL/min). Fraction 4 (26 -30 min; 400 mg) was further separated by repetitive preparative HPLC on Nucleodur 100 C18ec (5 FM, 250x21 mm, mobile phase A: 0.1% TFA in water; mobile phase B: 0.1% TFA in acetonitrile; gradient: start: 5% B, 1 min: 5% B, 40 min: 50% B, 45 min: 100% B, 50 min: 100% B; flow 20 mL/min) to afford compound 7 (1.4 mg), compound 4 (2.3 mg), compound 8 (0.5 mg) and compound 3 (0.8 mg). Fraction 5 (31 -32 min; 228 mg) was further separated by preparative HPLC on Nucleodur 100 C18ec (5 µM, 250x21 mm, mobile phase A: 0.1% TFA in water; mobile phase B: 0.1% TFA in acetonitrile; gradient: start: 5% B, 1 min: 5% B, 40 min: 50% B, 45 min: 100% B, 50 min: 100% B; flow 20 mL/min) to afford compound 1 (10 mg). Fraction 6 (33 -35 min) was further separated by repetative preparative HPLC on Nucleodur 100 C18ec (5 µM, 250x21 mm, mobile phase A: Q.1% TFA in water; mobile phase B: 0.1% TFA in acetonitrile; gradient: start: 5% B, 1 min: 5% B, 40 min: 50% B, 45 min: 100% B, 50 min: 100% B; flow 20 mL/min) to afford compound 5 (60 mg), compound 6 (4 mg) and compound 2 (3 mg).

### Example 4: Physicochemical data of compounds 1 - 10 / Structure elucidation

The structures of the compounds of examples 1 to 10 were determined by low- and high-resolution (ESI+/-) mass-spectrometry and one- and two-dimensional NMR spectrometry. Measurements were performed in [d6]-DMSO on a BRUKER AVANCE 500 spectrometer operating at 500.13 MHz proton frequency. All one-bond.¹H-¹³C connectivities were established by a heteronuclear multiple-quantum coherence (HMQC) experiment, ¹H-¹H connectivities were established by correlation spectrometry (COSY) and two-or three-bond ¹H-¹³C connectivities were established by heteronuclear multiple bond connectivity (HMBC) experiments.

| ex. | structure | chemical name |
|---|---|---|
| 1 | 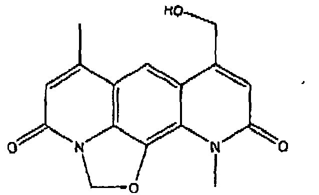 | nybomycin |
| 2 | 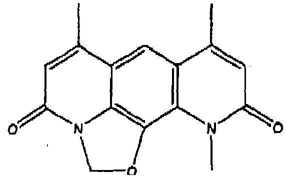 | deoxynybomycin |
| 3 | 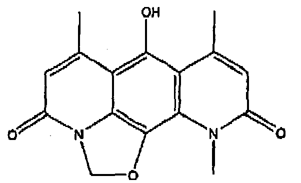 | 7-hydroxy-deoxynybomycin |
| 4 | 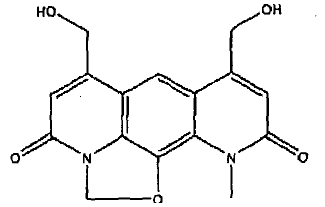 | 6'-hydroxynybomycin |
| 5 | 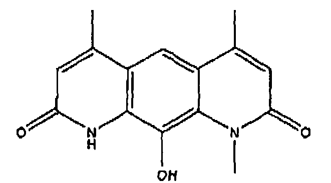 | 10-hydroxy-1,4,6-trimethylpyndo[3,2-g]quino-line-2,8(1*H*,9*H*)-dione |
| 6 | 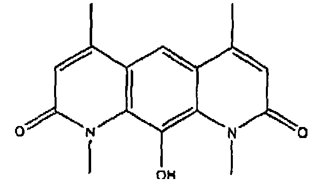 | 10-hydroxy-1,4,6,9-tetramethylpyrido[3,2-g]-quinoline-2,8(1*H*,9*H*)-dione |
| 7 | 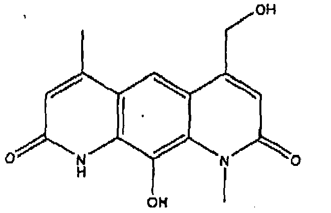 | 10-hydroxy-4-(hydroxymethyl)-1,6-dimethyl-pyrido[3,2-g]quinoline-2,8(1*H*,9*H*)-dione |
| 8 | 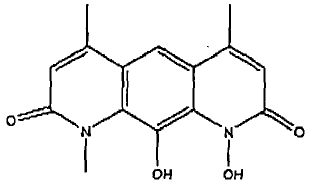 | 1,10-dihydroxy-4,6,9-trimethylpyrido[3,2-g]-quinoline-2,8(1*H*,9*H*)-dione |
| 9 | 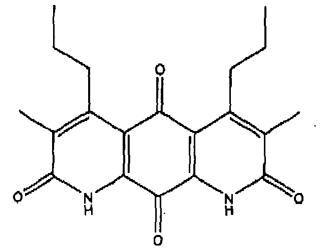 | 3,7-dimethyl-4,6-dipropylpyrido[3,2-g]-qujnoline-2,5,8,10(1*H,*9*H*)-tetraone; Diazaquinomycin A |
| 10 | 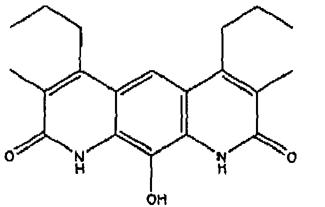 | 10-hydroxy-3,7-dimethyl-4,6-dipropylpyrido-[3,2-g]quinoline-2,8(1*H*,9*H)*-dione |

### Nybomycin 1

| | |
|---|---|
| HPLC: | Rₜ (Sys 1)= 7.48 min |
| LC-MS (ESI pos.): | *m*/*z* = 299 [M+H]⁺ |

¹H-NMR (500 MMz, DMSO-d₆): δ = 7.55 (s, 1H). 6.61 (s, 1 H), 6.41 (s, 1H), 6.40(s, 2H). 4.80(s, 2H). 3.82 (s, 3H), 2.55(s, 3H).

### Dexoxnybomycin 2

| | |
|---|---|
| HPLC: | Rₜ (Sys 1) = 6.32 min |
| LC-MS (ESI pos.): | *m*/*z* = 283 [M+H]⁺ |

¹H-NMR (500 MHz, DMSO-d₆). δ = 7.63 (s, 1H), 6.60 (s, 1H), 6.46 (s, 1H), 6.40 (s, 2H), 3.82 (s, 3H), 2.55 (s, 3H), 2.54 (s, 3H).

### 7-Hydroxy-deoxynybomycin 3

| | |
|---|---|
| HPLC: | Rₜ (Sys 1) = 6,32 min |
| LC-MS (ESI neg.): | *m*/*z* = 297 [M-H]⁻ |

¹H-NMR (500 MHz, DMSO-d₆): δ = 7.66 (s, br. OH), 6.82 (s, 1 H), 6.45 (s, 1H), 6.43 (s, 2H), 3.83 (s, 3H), 2.50 (s, 6H).

### 6'-Hydroxynybomycin 4

| | |
|---|---|
| HPLC: | Rₜ (Sys 1) = 6.33 min |
| LC-MS (ESI pos.): | *m*/*z =* 315 [M+H]⁺ |

¹H-NMR (500 MHz, DMSO-d₆): δ = 7.53 (s, 1H), 6.60 (s, 1H), 6.55 (s, 1 H), 6.42 (s, 2H), 4.82 (s, 2H), 4.79 (s, 2H), 3.81 (s, 3H).

### 10-hydroxy-1,4,6-trimethylpyrido[3,2-g]quinoline-2,8(1H,9H)-dione (5)

| | |
|---|---|
| HPLC: | Rₜ (Sys 1) = 8.15 min |
| LC-MS (ESI neg.): | *m*/*z* = 269 [M-H]⁻ |
| LC-MS (ESI pos.): | *m*/*z* = 271 [M+H]⁺ |

HR-MS: ESI+ ; *m*/*z* 271.1070 (calcd for C15H14N203 271.1083)
¹H-NMR (500 MHz, DMSO-d₆): δ = 7.62 (s, 1H, H-10), 6.42 (s, 1H, H-6), 6.38 (s, 1H, H-3), 3.81 (s, 3H, NCH₃), 2.48 (s, 3H, H-11), 2.45 (s, 3H, H-12);
¹³C-NMR (DMSO-d₆):δ = 162.47 (C-7), 161.76 (C-2). 146.72 (C-4), 148.14 (C-5), 133.25 (C-9a), 132.86 (C-8a), 120.69 (C-3), 119.84 (C-6), 119.16 (C-10a), 116.08 (C-4a), 115.35 (C-10), 35.12 (N-CH₃), 19.47 (C-12), 19.05 (C-11), C-9 n. det. in HMBC experiment.

### 10-hydroxy-1,4,6,9-tetramethylpyrido[3,2-g]quinoline-2,8(1H,9H)-dione (6)

| | |
|---|---|
| HPLC: | R₁ (Sys 1) = 8.98 min |
| LC-MS (ESI neg.) | *m*/*z* = 283 [M-H]⁻ |
| LC-MS (ESI pos.): | *m*/*z* = 285 [M+H]⁺ |

¹H-NMR (500 MHz, DMSO-d₆): δ = 7.59 (s, 1H, H-10). 6.46 (s, 2H, H-6, H-3), 3.72 (s, 6H, NCH₃), 2.47(s, 6H, H-11, H-12)
¹³C-NMR (DMSO-d₆): δ = 163.04 (C-2, C-7), 146.48 (C-4, C-5), 135.49 (C-8a, C-9a), 1 19.44 (C-3, C-6), 118.41 (C-4a, C-10a), 114.21 (C-10), 35.78 (N-CH₃), 18.72 (C-11, C-12), C-9 n. det. in HMBC experiment.

### 10-hydroxy-4-(hydroxymethyl)-1,6-dimethylplyrido[3,2-g]quinoline-2,8(1H,9H)-dione (7)

| | |
|---|---|
| HPLC: | Rₜ (Sys 1) = 6.58 min |
| LC-MS (ESI neg.): | *m*/z = 285 [M-H]⁻ |
| LC-MS (ESI pos.): | *m*/*z* = 287 [M+H]⁺ |

¹H-NMR (500 MHz, DMSO-d₆): δ = 7.56 (s, 1H, H-10), 6.57 (s, 1H, H-3), 6.38 (s, 1H, H-6), 5.49 (s, br. 1H, OH), 4.79 (s, 2H, H-11), 3.84 (s, 3H, NCH₃), 2.46 (s, 3H, H-12)
¹³C-NMR (DMSO-d₆): δ = 163.38 (C-2), 150.75 (C-4). 149.02 (C-5),133.77 (C-9a), 130.25 (C-8a), 129.79 (C-6), 1 16.14 (C-3), 116.79 (C-10a), 1 16.37 (C-4a), 114.18 (C-10), 34.98 (N-CH₃), 19.18 (C-12), C-7 and C-9 n. det. in HMBC experiment.

### 1,10-dihydroxy-4,6,9-trimethylpyrido[3,2-g]quinoline-2,8(1H,9H)-dione (8)

| | |
|---|---|
| HPLC: | Rₜ (Sys 1) = 6.53 min |
| LC-MS (ESI neg.): | *m*/*z* = 285 [M-H]⁻ |
| LC-MS (ESI pos.): | *m*/*z* = 287 [M+H]⁺ |

HR-MS: *m*/*z* 285.088 (calcd for C₁₅H₁₃N₂O₄ 285.0875)
¹H-NMR (500 MHz, DMSO-d₆): δ = 7.65 (s, 1H, H-10), 6.79 (s, 1H, H-3), 6.41 (s, 1H, H-6), 3.79 (s, 3H, NCH₃), 2.36 (s, 3H, H-11), 2.51 (s, 3H. H-12)
¹³C-NMR (DMSO-d₆): δ = 163.38 (C-7), 149.28 (C-4), 148.65 (C-5), 1 18.62 (C-3), 121.21 (C-6), 1 13.68 (C-10a), 115.79 (C-4a), 114.18 (C-10). 32.46 (N-CH₃). 19.14 (C-11), 18.08 (C-12), C-7, C-9a, C-8a and C-9 n. det. in HMBC experiment.

### 3,7-dimethyl-4,6-dipropylpyrido[3,2-g]quinoline-2,5,8,10(1H,9H)-tetraone (9)

| | |
|---|---|
| HPLC: | Rₜ (Sys 1) = 13.26 min |
| LC-MS (ESI neg.): | *m*/*z* = 353 [M-H]⁻ |
| LC-MS (ESI pos.): | *m*/*z* = 355 [M+H]⁺ |

HR-MS: ESI- *m*/*z* 353.1498 (calcd for C₂₀H₂₁N₂O₄ 353.1501)
¹H-NMR (500 MHz, DMSO-d₆): δ = 1.01 (t, J = 7.25 Hz, 6H, CH₃CH₂CH₂), 1.50 (m, 4H, CH₃CH₂CW₂), 2.12 (s, 6H), 2.94 (t, J= 7.6 Hz, 4H, CH₃CH₂CH₂).
The data complain with the literature (Omura 1983)

### 10-hydroxy-3,7-dimethyl-4,6-dipropylpyrido[3,2-g]quinoline-2,8(1H,9H)-dione (10)

| | |
|---|---|
| HPLC: | Rₜ (Sys 1) = 12.79 mi |
| LC-MS (ESI neg.): | *m*/*z* = 339 [M-H]⁻, 679 [2M-H]⁻ |
| LC-MS (ESI pos.): | *m*/*z* = 341 [M+H]⁺, 681 [2M+H]⁺; |

HR-MS: *m*/*z* 339.1691 (calcd for C₂₀H₂₃N₂O₃ = 339.1709)
¹H-NMR (500 MHz, DMSO-d₆): δ = 1.06 (t, J 7.2 Hz, 6H, CH₃CH₂CH₂), 1.61 (m, 4H, CH₃CH₂CH₂). 2.10 (s, 6H), 2.87 (t, J = 7.6 Hz, 4H, CH₃CH₂CH₂), 7.49 (s, 1 H)
¹³C-NMR (DMSO-d₆): δ =161.8 (C-2, C-7), 147.5 (C-4, C-5), 124.2 (C-3, C-6), 115.35 (C-4a, C-10a), 126.38 (C-10), 31.81 (C-12, C-15), 22.22 (C-13, C-16). 14.97 (C-14, C-17). 13.46 (C-11, C-18), C-9a. C-8a and C-9 n. det. in HMBC experiment.

### Example 5: Synthesis

### Anisol-2,6-diammonium chloride (11)

22.0 g (111 mmol) of 2,6-Dinitroanisol were dissolved in 1.5 L of MeOH-THF (5:1). 1N aqueous hydrochloric acid was added (20.2 mL, 2.2 eq). After addition of 4.40 g (20% w/w) of Pd/C (10%), the mixture was hydrated at rt and normal pressure. Complete conversion was achieved after 2d. The mixture was filtrated and the eluate dried *in vacuo* to obtain 22.9 g (98%) of the title compound

### 2.6-Diamino-3,5-dibromo-anisol (12)

Dichloromethane (592 mL) and triethylamine (14.2 mL, 102 mmol, 6 eq) were added to 2.6 g (17 mmol) of 11. *N*-Bromosuccinimid (6.68 g, 37.4 mmol, 2.2 eq) was added in small portions within 3h at rt, afterwards the mixture was stirred for another 2h. After removing the solvent *in vacuo*, the residue was re-dissolved in dichloromethane and filtrated over silica gel. Again the solvent was removed and the residue dissolved in ethyl acetate. Charcoal was added for 30 min at 50 °C, afterwards the mixture was filtrated over silica gel and evaporated to dryness. Column chromatography (silica gel, cyclohexane-ethyl acetate 3:1) yielded 3.5 g of the title compound (69%).

### (2E,2'E)-Diethyl 3,3'-(4,6-diamino-5-methoxy-1,3-phenylene)dibut-2-enoate (13)

5 g of 12 (16.9 mmol) were dissolved in DMF (47 mL) under argon atmosphere. Palladium(II)acetate 99.9% (1.52 g, 6.76 mmol, 0.4 eq). Tritolylphosphin (6.17 g, 20.3 mmol, 1.2 eq), Crotonic acid ethyl ester (208 mL, 1672 mmol, 99 eq) and triethylamine (23.6 mL, 169 mmol, 10 eq) were added, while the apparatus was degassed and refilled with argon several times. The mixture was stirred at 110 °C for 4h and afterwards evaporated to dryness. The residue was filtrated over kieselgur (DCM-MeOH 10:1).
a) Column chromatography (silica gel, cyclohexane-ethyl acetate 3:1 → 2:1) yielded 2.5 g of compound 13 (26%). After eluation of the column with DCM-MeOH (20:1), 3.0 g of an oily fraction containing compound 14 were obtained. Purification of 14 was achieved by only by preparative HPLC.
b) Alternatively, the crude mixture could be used for the next step after kieselgur filtration.

### (E)-Ethyl 3-(7-amino-8-methoxy-4-methyl-2-oxo-1.2-dihydroquinolin-6-yl)but-2-enoate (14)

Compound 13 (1.7 g, 4.7 mmol) was dissolved in pure acetic acid and stirred over night at rt. After evaporation, 1.4 g (118%) of the title compound were obtained.
Altematively, the reaction was done with the crude mixture as described above b).

### 10-Methoxy-1,4,6-trimethvjpvrido[3.2-g]quinoline-2,8(1H,9H)-dione (15)

The reaction was performed under argon atmosphere at rt, using a water bath for temperature control. Sodium hydride in mineral oil (4 mmo, 2.5 eq) was added stepwise to a mixture of 500 mg (1.58 mmol) of compound 14 and 7 mL DMF. The mixture was stirred for 2h and afterwards evaporated to dryness. Purification was achieved by preparative HPLC to obtain 209 mg (47%) of the crystalline title compound.

### 10-Hydroxy-1,4,6-trimethylpyrido[3,2-g]quinoline-2.8(1H,9H)-dione (5)

Compound 15 (110 mg, 0.39 mmol) was dissolved in 48% hydrobromic acid (100 mL) and stirred at 140 °C for 3h. After evaporating to dryness, toluene was added and removed *in vacuo*. Triethylamine (0.11 mL, 2 eq) was given to the residue under supersonic treatment. Evaporation and washing of the residue with 15 mL dichloromethane yielded the preliminary product (108 mg, 103%), which was further purified by washing with 10 mL hot acetonitrile-water solution (3:1). Finally, 85 mg (81%) of the pure title compound were obtained.

### Example 6: Evaluation of physiological activity

The *in vitro* effect of the compounds according to the invention can be demonstrated in the following assays:

### Assay #1: CDC25C Enzyme Assay

An enzyme preparation was used with a total protein concentration of 11.6 mg protein/mL. Gel analysis of the protein solution indicated a CDC25C protein content of ca. 30%. The assay principle was hydrolysis of an artificial fluorogenic substrate of CDC25C, 3-*0-*methyl-fluorescein monophosphate (OMFP). Upon hydrolysis of this phosphate ester by CDC25C, the product 3-*0-*methyl-fluorescein exhibits fluorescence at 535 nm (λₑₓ=485 nm). The assay was performed in 96 well microtiter plates (Greiner 96 well black FIA plates) at ambient temperature.

The additions to the wells were as follows:
- 1: Addition of 4 µl of a DMSO solution of the test compound; controls received 4 µl of DMSO instead
- 2: Addition of 76 µl assay buffer (assay buffer: 100 mM Tris pH 8.2, 40 mM NaCl. 5 mM DTT, 20% glycerol)
- 3: Addition of 10 µl CDC25 C (CDC25 C in assay buffer; protein final concentration 1.1 µg/mL)
- 4: Start of reaction by addition of 10 µl OMFP (substrate in assay buffer; final concentration of OMFP in assay: 90 µM )

Ortho-vanadate was used as a reference inhibitor. Background controls contained buffer, substrate and DMSO, but no enzyme. The microtiter plates were analyzed using a Tecan Ultra fluorescence reader. For data evaluation, background fluorescence was subtracted from total fluorescence intensity after a standard assay run time of 20 minutes

**Table #4: Results assay #1**

| example | IC₅₀ [µM] |
|---|---|
| 1 comparative | > 20 |
| 2 comparative | > 10 |
| 3 comparative | 5 |
| 4 comparative | 14 |
| 5 | 0.18 |
| 6 comparative | 14.5 |
| 7 | 0.93 |
| 8 | 0.52 |
| 9 comparative | 225 |
| 10 | 0.85 |

The results of *in vitro* cell assays against a leukemia cell line (P388) suprisingly revealed that the N-mono-demethyl compounds according to invention (5, 7, 8 and 10) exhibit a much higher potential as inhibitors of CDC25 than the compounds of the comparative examples (1, 2, 3, 4, 6 and 9).

The compound of example 5 was thoroughly characterized towards its selectivity for CDC25 sub-types A, B, and C. After 20 minutes of pre-incubation, in inhibition assays against CDC25 A, B, and C the compound revealed IC₅₀ values of 150 nM against CDC25A; 230 nM against CDC25B and 180 nM against CDC25C, respectively.

These findings indicate a comparable potency against all CDC25 sub-types. All CDC25 sub-types control cell cycle propagation at different but always essential steps. Their respective overexpression has been reported from many different types of human tumors and it has been correlated with bad prognosis.

Specificity for one of the three human CDC25 phosphatases is still a matter of debate. Most of the CDC25 inhibitors identified yet do not differentiate inbetween the CDC25 sub-types. It is believed that a broad CDC25 inhibitory effect is a more efficient way to control cell proliferation through the targeting of multiple cell cycle essential steps (Brezak MC et al., Mol Cancer Ther 2005; 4(9), 1378-1387).

Additionally to its characterization on CDC25 sub-types, the compound of example 5 has been characterized in counter-screens against other phosphatases to demonstrate selectivity. Screens performed against PTP1 B and LAR (PTPRF) revealed 13- and 100-fold selectivity of the compound of example 5 for CDC25 subtypes in comparison to PTP1 B and LAR.

Thus, the compounds of this invention clearly differentiate between different phosphatases, i.e., have a high selectivity against CDC25 without simultaneously inhibiting PTP1 B or LAR. Further, the compounds of this invention do not significantly differentiate between the individual sub-types of CDC25 (A, B and C), i.e., exhibit a broad CDC25 inhibitory effect.

### Assay #2: Cell proliferation Assay

Used cell lines: HCT116; DU145; A549, H460
In a 96-well plate, 3000 cells/well were plated in 100 µl DMEM containing 10% FBS, 10 mM HEPES and 1% Penicllin/Streptomycin. To each well 50 µl of additional growth media containing the compound with 0.1% DMSO was added. Cells were grown for 72 hrs. at 37°C. 20 µl of Alamar Blue reagent was added and incubated for 3-5 hours until color develops. Plates were read in a SpectraMax Gemini (Molecular Devices, CA) with 544 nm excitation and 590 nm emission wavelength.

**Table #5: Results assay #2**

| compounds | HCT116; IC₅₀ | DU145; IC₅₀ | A549; IC₅₀ | H460; IC₅₀ |
|---|---|---|---|---|
| 1 comparative | 730 nM | 720 nM | n.d. | 230 nM |
| 5 | 100 nM | 100 nM | 100 nM | < 300 nM |
| 6 comparative | 3,000 nM | 3,000 nM | < 10,000 nM | < 10,000 nM |

Compound #5 has demonstrated anti-proliferative efficacy in several cell lines tested *in vitro.* Furthermore, results from assay #2 demonstrate superior *in vitro* potency of compound 5 versus comparative compounds # 1 and #6.
These findings indicate that compound #5 and its derivatives might serve as potent therapeutics for cancer therapy.

## Claims

1. A compound of general formula (I) 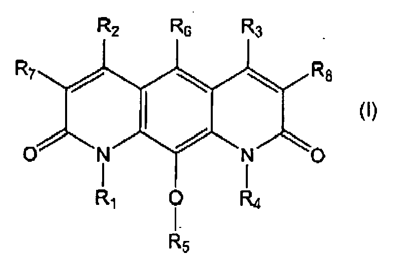 wherein
R₁ is -H, -C₁₋₈-alkyl, -C(=O)-C₁₋₈-alkyl, -C(=O)-O-C₁₋₈-alkyl, -C(=O)-NH-C₁₋₈-alkyl, -C(=O)-N(d₁₋₈-alkyl)₂, -F, -Cl, -Br, -I, -NO₂, -CN, -CHO, -CO₂H, -OH, -O-C₁₋₈-alkyl, -O-C(=O)-C₁₋₈-alkyl, -SH, -S-C₁₋₈-alkyl, -NH₂, -NH-C₁₋₈-alkyl, -NH-C(=O)-C₁₋₈-alkyl, or -N(C₁₋₈-alkyl)₂;
R₂ and R₃ are independently of each other -H, -C₁₋₈-alkyl, -phenyl, -C₁₋₈-alkylphenyl, -C(=O)-C₁₋₈-alkyl, -C(=O)-O-C₁₋₈-alkyl, -C(=O)-NH-C₁₋₈-alkyl, -C(=O)-N(C₁₋₈-alkyl)₂, -F, -Cl, -Br, -I, -NO₂. -CN, -CHO, -CO₂H, -OH, -O-C₁₋₈-alkyl, -O-phenyl, -O-C₁₋₈-alkyl-phenyl, -O-C(=O)-C₁₋₈-alkyl, -SH, -S-C₁₋₈-alkyl, -NH₂, -NH-C₁₋₈-alkyl, -NH-C(=O)-C₁₋₈-alkyl, or -N(C₁₋₈-alkyl)₂;
R₄ is -H, -C₁₋₈-alkyl, -phenyl, -C₁₋₈-arkyl-phenyl. -C(=O)-C₁₋₆-alkyl, -C(=O)-O-C₁₋₈-alkyl, -C(=O)-NH-C₁₋₈-alkyl, -C(=O)-N(C₁₋₈-alkyl)₂, -F, -Cl. -Br, -1, -NO₂, -CN, -CHO, -CO₂H, -OH, -O-C₁₋₈-alkyl, -O-C(=O)-C₁₋₈-alkyl, -SH, -S-C₁₋₈-alkyl, -NH₂, -NH-C₁₋₈-alkyl, -NH-C(=O)-C₁₋₈-alkyl, or -N(C₁₋₈-alkyl)₂;
R₅ is -H, -C₁₋₈-alkyl, -phenyl, -C₁₋₈-alkyl-phenyl, -C(=O)-C₁₋₈-alkyl. -C(=O)-O-C₁₋₈-alkyl, -C(=O)-NH-C₁₋₈-alkyl, or -C(=O)-N(C₁₋₈-alkyl)₂;
R₆ is -H, -C₁₋₈-alkyl, -phenyl, -C₁₋₈-alkyl-phenyl, -C(=O)-C₁₋₈-alkyl. -C(=O)-O-C₁₋₈-alkyl, -C(=O)-NH-C₁₋₈-alkyl, -C(=O)-N(C₁₋₈-alkyl)₂, -F, -Cl, -Br, -I, -NO₂, -CN, -CHO, -CO₂H, -OH. -O-C₁₋₈-alkyl, -O-phenyl, -O-C₁₋₈-alkyl-phenyl, -O-C(=O)-C₁₋₈-alkyl, -SH, -S-C₁₋₈-alkyl, -NH₂, -NH-C₁₋₈-alkyl, -NH-C(=O)-C₁₋₈-alkyl, or -N(C₁₋₈-alkyl)₂;
R₇ and R₈ are idenpendently of each other -H, -C₁₋₈-alkyl, -C(=O)-C₁₋₈-alkyl, -C(=O)-O-C₁₋₈-alkyl, -C(=O)-NH-C₁₋₈-alkyl, -C(=O)-N(C₁₋₈-alkyl)₂, -F, -Cl, -Br, -I, -NO₂, -CN, -CHO, -CO₂H, -OH, -O-C₁₋₈-alkyl, -O-phenyl, -O-C₁₋₈-alkyl-phenyl, -O-C(=O)-C₁₋₈-alkyl, -SH, -S-C₁₋₈-alkyl. -NH₂, -NH-C₁₋₈-alkyl, -NH-C(=O)-C₁₋₈-alkyl, or -N(C₁₋₈-alkyl)₂;
or R₂ together with R₆ and/or R₂ together with R₇ and/or R₃ together with R₆ and/or R₃ together with R₆ independently of each other forms a five- to seven-membered saturated or unsaturated hydrocarbonaceous ring, where one or two C ring atoms are optionally replaced by heteroring atoms independently of each other selected from the group consisting of N, O and S, and where said hydrocarbonaceous ring is unsubstituted or contains one or two substituents independently of each other selected from the group consisting of -C₁₋₈-alkyl, -C(=O)-C₁₋₈-alkyl, -C(=O)-O-C₁₋₈-alkyl, -C(=O)-NH-C₁₋₈-alkyl. -C(=O)-N(C₁₋₈-alkyl)₂, -F, -Cl, -Br, -I, -NO₂, -CN, -CHO, -CO₂H, -OH, -O-C₁₋₈-alkyl, -O-C(=O)-C₁₋₈-alkyl. -SH, -S-C₁₋₈-alkyl, -NH₂, -NH-C₁₋₈-alkyl, -NH-C(=O)-C₁₋₈-alkyl, or -N(C₁₋₈-alkyl)₂;
with the proviso that when R₅ is -H, R₆ is not -OH; and that R₁, R₂, R₃ and R₄ are not simultaneously -CH₃;
or the physiologically acceptable salts thereof,
for use as a medicament.

2. The compound according to claim 1, wherein R₁ is -H or -CH₃; R₄ is -H or -OH; and R₅ is -H.

3. The compound according to claim 1 or 2, wherein at least two residues selected from R₁, R₂, R₃ and R₄ are identical.

4. The compound according to any of the preceding claims, wherein R₂ and R₃ are independently of each other -H, -OH, -C₁₋₈-alkyl, or -C₁₋₈-alkyl-OH.

5. The compound according to any of the preceding claims, wherein R₆ is -H, -F, -Cl, -Br, -C₁₋₈-alkyl, -CF₃, -O-C₁₋₈-alkyl, or -C₁₋₈-alkyl-OH.

6. The compound according to any of the preceding claims, wherein R₇ and R₈ are idenpendently of each other -H, or -C₁₋₈-alkyl.

7. A pharmaceutical composition comprising a compound of general formula (I) as defined in any of claims 1 to 6 and a physiologically acceptable carrier.

8. Use of a compound of general formula (I) as defined in any of claims 1 to 6 for the manufacture of a medicament for treating cancer.

9. The use according to claim 8, wherein the cancer is selected from the group consisting of malignant melanoma, malignant lymphoma, lung cancer, esophageal cancer, gastric cancer, large-bowel cancer, rectal cancer, colon cancer, urinary tract tumor, gallbladder cancer, bile duct cancer, biliary tract cancer, breast cancer, liver cancer, pancreatic cancer, testis tumor, maxillary cancer, tongue cancer, lip cancer, oral cavity cancer, pharynx cancer, larynx cancer, ovarian cancer, uterine cancer, prostate cancer, thyroid cancer, brain tumor, Kaposi's sarcoma, angioma, leukemia, polycythemia vera, neuroblastoma, retinoblastoma, myeloma, bladder tumor, sarcoma, osteosarcoma, myosarcoma, skin cancer, basal-cell carcinoma, cutaneous appendage tumor, skin metastatic cancer, and skin melanoma.

10. A method for treating cancer comprising the administration of a physiologically effective amount of a compound of general formula (I) as defined in any of claims 1 to 6.

11. The method of claim 10, wherein the cancer is selected from the group consisting of malignant melanoma, malignant lymphoma, lung cancer, esophageal cancer, gastric cancer, large-bowel cancer, rectal cancer, colon cancer, urinary tract tumor, gallbladder cancer, bile duct cancer, biliary tract cancer, breast cancer, liver cancer, pancreatic cancer, testis tumor, maxillary cancer, tongue cancer, lip cancer, oral cavity cancer, pharynx cancer, larynx cancer, ovarian cancer, uterine cancer, prostate cancer, thyroid cancer, brain tumor, Kaposi's sarcoma, angioma, leukemia, polycythemia vera, neuroblastoma, retinoblastoma, myeloma, bladder tumor, sarcoma, osteosarcoma, myosarcoma, skin cancer, basal-cell carcinoma, cutaneous appendage tumor, skin metastatic cancer, and skin melanoma.

12. A compound of general formula (I) as defined in any of claims 1 to 6, with the proviso that
(iv) when R₁, R₂ and R₃ are each -CH₃, and R₅, R₆, R₇ and R₈ are each -H; or
(v) when R₁, R₂, R₃ and R₅ are each -CH₃, and R₆, R₇ and R₈ are each -H; or
(vi) when R₂, R₃ and R₅ are each -CH₃, and R₁, R₆, R₇, and R₈ are each -H;
R₄ is not -H.

13. The compound according to claim 12, selected from the group consisting of
10-hydroxy-4-(hydroxymethyl)-1,6-dimethylpyrido[3,2-g]quinoline-2,8(1*H*,9*H*)-dione;
10-hydroxy-3,7-dimethyl-4,6-dipropylpyrido[3,2-g]quinoline-2,8(1*H,*9*H*)-dione: and
1,10-dihydroxy-4,6,9-trimethylpyrido[3,2-g]quinoline-2,8(1*H*,9*H*)-dione
and the physiologically acceptable salts thereof.

14. A process for the synthesis of a compound of general formula (I) as defined in claim 12 or 13, comprising the step of a double Heck Reaction.

15. A microorganism with the designation number DSM 18699, DSM 18700, DSM 18701, DSM 18698 or DSM 18697 **characterised by** its 16S rDNA sequences as presented in the sequence listing.


Sequence listing

<110>	InterMed Discovery GmbH
<120>	CDC25 Inhibitors
<160>	5

<210>	001
<211>	438
<212>	DNA
<213>	Streptomyces spec., BC16304
<400>	001
tgggcaatct gcccttcact ctgggacaaa accctggaaa cggggtctaa taccggatga
tacttccact cgcataaata aaaattgaaa gctccggcgg tgaaggatga gcccgcggcc
tatcagcttg ttggtgaggt agtggctcac caaggcgacg acgggtagcc ggcctgagag
ggcgaccggc cacactggga ctgagacacg gcccagactc ctacgggagg cagcagtggg
gaatattgca caatgggcga aagcctgatg cagcgacgcc gcgtgaggga tgacggcctt
cgggttgtaa acctctttca gcagggaaga agcgaaagtg acggtacctg cagaagaagc
gccggctaac tacgtgccag cagccgcggt aatacgtagg gcgcaagctt gtccggaatt
attgggcgta aagagctc

<210>	002
<211>	443
<212>	DNA
<213>	Streptomyces spec., BC20243
<400>	002
tgggcaactg ccctgcactc tgggacaaag ccctggaaac ggggtctaat accggatact
gatccgtctg ggcatccaga tggttcgaaa gctccggcgg tgcaggatga gcccgcggcc
tatcagcttg ttggtgaggt agtggctcac caaggcgacg acgggtagcc ggcctgagag
ggcgaccaac cacactggga ctgagacacg gcccagactc ctacgggagg cagcagtggg
aatattgcac aatgggcgaa agcctgatgc agcgacgccg cgtgagggat gacggccttc
gggttgtaaa cctctttcag cagggaagaa gcgaaagtga cggtacctgc agaagaagcg
ccggctaact acgtgccaca gccgcggtaa tacgtagggc gcgagcgttg tccggaatta
ttggcgtaaa gagctcgtag gcg

<210>	003
<211>	426
<212>	DNA
<213>	Streptomyces spec., BC25486
<400>	003
gggcaatctg cccttcactc tgggacacgc cctggaaacg gggtctaata ccggatacna
ccaatttggg gcatccgttg agtgtggaaa gctccggcgg tgaaggatga gcccgcggcc
tatcagcttg ttggtgaggt aacggctcac caaggcgacg acgggtagcc ggcctgagag
ggcgaccggc cacactggga ctgagacacg gcccagactc ctacgggagg cagcagtggg
gaatattgca caatgggcga aagcctgatg cagcgacgcc gcgtgaggga tgacggcctt
cgggttgtaa acctctttca gcagggaaga agcgaaagtg acggtacctg cagaagaagc
gccggctaac tacgttccag cagccgcggt aatacgtagg cgcgagcgtt gtccggatta
ttggcg

<210>	004
<211>	408
<212>	DNA
<213>	Streptomyces spec., BC25518
<400>	004
gggcaatctg cccttcactc tgggacangc cctggaaacg gggtctaata ccggatgata
cttccactcg catgggtggg ggttgaaagc tccggcggtg agggatgagc ccgcggccta

tcagcttgtt ggtgaggtag aggctcacca aggcgacgac gggtagccgg cctgagaggg
cgaccggcca cactgggact gagacacgcc cagactccta cgggaggcag cagtggggaa
tattgcacaa tgggcgcaag cttgatgcag cgacgccgcg tgagggatga cggccttcgg
gttgtaaacc tctttcagca gggaagaagc gaaagtgacg gtacctgcag aagaagcgcc
ggctaactac gtgccagcag ccggtaatac gtagggcgca aggttgtc

<210> 005
<211> 433
<212> DNA
<213> Streptomyces spec., BC28272
<400> 005
gggcaatctg ccctgcactc tgggacagcc ctggaaacgg ggtctaatac cggatatgag
cctcttccgc atggttgagg ttgtaaagct ccggcggtgc aggatgagcc cgcggcctat
cagcttgttg gtgaggtaac ggctcaccaa ggcgacgacg ggtagccggc ctgagagggc
gaccggccac actgggactg agacacggcc cagactccta cgggaggcag cagtggggaa
tattgcacaa tgggcgaaag cctgatgcag cgacgccgcg tgagggatga cggccttcgg
gttgtaaacc tctttcagca gggaagaagc gaaagtgacg gtaggtgcag aagaagcgcc
ggctaactac gtgccagcag ccgcggtaat acgtagggcg caaggcttgt ccggaattat
tggcgtaaga gct